# EUROPEAN PATENT SPECIFICATION

(11) **EP 3 743 099 B1**
(45) Date of publication and mention of the grant of the patent: **24.06.2026**
(21) Application number: 19701656.1
(22) Date of filing: 25.01.2019
(51) Int. Cl.: A61K 39/00, A61P 35/00

(54) **DENDRITIC CELL VACCINATION IN PARALLEL TO CHEMOTHERAPY**
IMPFUNG MIT DENDRITISCHEN ZELLEN ZUSAMMEN MIT DER CHEMOTHERAPIE
VACCINATION À BASE DE CELLULES DENDRITIQUES PARALLÈLEMENT À UNE CHIMIOTHÉRAPIE

(30) Priority: 25.01.2018 EP 18153415
(43) Date of publication of application: 02.12.2020
(73) Proprietor: Excoso a.s., 190 00 Prague 9 (CZ)
(72) Inventor: SPISEK, Radek, 15 500 Prague 8 (CZ); BARTUNKOVA, Jirina, 13 000 Prague 3 (CZ)
(74) Representative: Maiwald GmbH
(86) International application number: PCT/EP2019/051842
(87) International publication number: WO 2019/145471

(56) References cited:
- CHU C S ET AL: "Phase I/II randomized trial of dendritic cell vaccination with or without cyclophosphamide for consolidation therapy of advanced ovarian cancer in first or second remission", CANCER IMMUNOLOGY, IMMUNOTHERAPY, vol. 61, no. 5, 22 October 2011 (2011-10-22), pages 629 - 641, XP035047692, ISSN: 1432-0851, DOI: 10.1007/S00262-011-1081-8
- MEL�O ALICE: "SOTIO Enrolls First Patient in Another Phase 2 Trial of Its Ovarian Cancer Immunotherapy", OVARIAN CANCER NEWS TODAY, 29 November 2017 (2017-11-29), XP93225531, Retrieved from the Internet <URL:https://ovariancancernewstoday.com/2017/11/29/sotio-starts-another-phase-2-trial-of-its-ovarian-cancer-immunotherapy/>
- DONG WEI ET AL: "Combination of DC Vaccine and Conventional Chemotherapeutics", ANTI-CANCER AGENTS IN MEDICINAL CHEMISTRY, vol. 16, no. 5, 17 March 2016 (2016-03-17), pages 558 - 567, XP055485365, ISSN: 1871-5206, DOI: 10.2174/1871520615666150907094139
- BLOY N ET AL: "Trial watch: Dendritic cell-based anticancer therapy", ONCOIMMUNOLOGY, vol. 3, no. 11, E963424, 1 November 2014 (2014-11-01), pages 1 - 16, XP009184345, ISSN: 2162-4011, DOI: 10.4161/21624011.2014.963424
- TANAKA F ET AL: "Intratumoral injection of dendritic cells after treatment of anticancer drugs induces tumor-specific antitumor effect in vivo", INTERNATIONAL JOURNAL OF CANCER, vol. 101, no. 3, 20 September 2002 (2002-09-20), pages 265 - 269, XP002425822, ISSN: 0020-7136, DOI: 10.1002/IJC.10597
- KOIDO S ET AL: "Treatment with Chemotherapy and Dendritic Cells Pulsed with Multiple Wilms' Tumor 1 (WT1)-Specific MHC Class I/II-Restricted Epitopes for Pancreatic Cancer", CLINICAL CANCER RESEARCH, vol. 20, no. 16, 15 August 2014 (2014-08-15), pages 4228 - 4239, XP002780582, DOI: 10.1158/1078-0432.CCR-14-0314
- ROZKOVA D ET AL: "FOCUS on FOCIS: Combined chemo-immunotherapy for the treatment of hormone-refractory metastatic prostate cancer", CLINICAL IMMUNOLOGY, vol. 131, no. 1, April 2009 (2009-04-01), pages 1 - 10, XP026063794, ISSN: 1521-6616, [retrieved on 20090208], DOI: 10.1016/J.CLIM.2009.01.001
- LESTERHUIS W J ET AL: "A pilot study on the immunogenicity of dendritic cell vaccination during adjuvant oxaliplatin/capecitabine chemotherapy in colon cancer patients", BRITISH JOURNAL OF CANCER, vol. 103, no. 9, 5 October 2010 (2010-10-05), pages 1415 - 1421, XP055485368, ISSN: 0007-0920, DOI: 10.1038/sj.bjc.6605935
- LIU YANDI ET AL: "DC vaccine therapy combined concurrently with oral capecitabine in metastatic colorectal cancer patients", HEPATO-GASTROENTEROLOGY, vol. 60, no. 121, 31 December 2012 (2012-12-31), pages 23 - 27, XP009506157, ISSN: 0172-6390
- BAUER C ET AL: "Dendritic cell-based vaccination of patients with advanced pancreatic carcinoma: results of a pilot study", CANCER IMMUNOLOGY, IMMUNOTHERAPY, vol. 60, no. 8, 6 May 2011 (2011-05-06), pages 1097 - 1107, XP019929202, ISSN: 1432-0851, DOI: 10.1007/S00262-011-1023-5
- KOBAYASHI M ET AL: "Prognostic factors related to add-on dendritic cell vaccines on patients with inoperable pancreatic cancer receiving chemotherapy: a multicenter analysis", CANCER IMMUNOLOGY, IMMUNOTHERAPY, vol. 63, no. 8, 29 April 2014 (2014-04-29), pages 797 - 806, XP055485392, ISSN: 0340-7004, DOI: 10.1007/s00262-014-1554-7
- ELLEBAEK E ET AL: "Metastatic melanoma patients treated with dendritic cell vaccination, Interleukin-2 and metronomic cyclophosphamide: results from a phase II trial", CANCER IMMUNOLOGY, IMMUNOTHERAPY, vol. 61, no. 10, 20 March 2012 (2012-03-20), pages 1791 - 1804, XP035115266, ISSN: 1432-0851, DOI: 10.1007/S00262-012-1242-4
- MIKYSKOVÁ R ET AL: "Dendritic cells pulsed with tumor cells killed by high hydrostatic pressure induce strong immune responses and display therapeutic effects both in murine TC-1 and TRAMP-C2 tumors when combined with docetaxel chemotherapy", INTERNATIONAL JOURNAL OF ONCOLOGY, vol. 48, no. 3, March 2016 (2016-03-01), pages 953 - 964, XP055485852, ISSN: 1019-6439, DOI: 10.3892/ijo.2015.3314
- PODRAZIL M ET AL: "Phase I/II clinical trial of dendritic-cell based immunotherapy (DCVAC/PCa) combined with chemotherapy in patients with metastatic, castration-resistant prostate cancer", ONCOTARGET, vol. 6, no. 20, 20 July 2015 (2015-07-20), pages 18192 - 18205, XP055485867, DOI: 10.18632/oncotarget.4145
- FUCIKOVA J ET AL: "Phase I/II trial of dendritic cell-based active cellular immunotherapy with DCVAC/PCa in patients with rising PSA after primary prostatectomy or salvage radiotherapy for the treatment of prostate cancer", CANCER IMMUNOLOGY, IMMUNOTHERAPY, vol. 67, no. 1, 25 September 2017 (2017-09-25), pages 89 - 100, XP036390276, ISSN: 0340-7004, [retrieved on 20170925], DOI: 10.1007/S00262-017-2068-X

## Description

The immune system is a powerful tool that, if better harnessed, could enhance the efficacy of cytotoxic agents and improve outcomes for cancer sufferers. Vaccination is hoped to be an effective method of harnessing the immune system to eliminate cancer cells. Its activity is mostly dependent on antigen-specific CD8⁺ T cells that generate cytotoxic T lymphocytes (CTLs) to reject cancer (Palucka and Banchereau 2013). The immune system components necessary for the induction of such CD8⁺ T cells include the presentation of antigens by appropriate antigen-presenting cells (APCs). Dendritic cells (DCs) are the most efficient APCs, and are an essential component of vaccination through their capacity to capture, process, and present antigens to T cells (Banchereau and Steinman 1998). Multiple cancer vaccination strategies based on DCs have been developed (Galluzzi et al. 2012). One strategy is to culture patient-derived (autologous) monocytes with specific cytokine combinations, load them with tumor-associated antigen (TAAs) ex-vivo in the presence of adjuvants to promote DC maturation and eventually re-administer them into the patient. Other strategies are to deliver TAAs to DCs in vivo or approaches based on DC-derived exosomes (Galluzzi et al. 2012). These strategies have been tested in multiple clinical trials, ranging over a large variety of cancers (Galluzzi et al. 2012; Vacchelli et al. 2013; Bloy et al. 2014; Garg et al. 2017). However, these DC vaccine tested in clinical trials have demonstrated relatively poor therapeutic efficacy. Poor efficacy may be related to many reasons, such as maturation agents used during the DC vaccine preparation, limited immunogenicity of the TAAs loaded upon the DCs, sub-optimal localization in-vivo of the administered DCs, and/or formulation of the DC vaccines (Galluzzi et al. 2012). Poor efficacy may also be related to clinical trial designs, where the treatment usually includes standard of care chemotherapy. It has been shown that after chemotherapy, immune system recovery may be slower than believed (Verma et al. 2016), further highlighting the unpredictability of a DC vaccine-chemotherapy combination treatment.

On the other hand, despite great efforts to optimize first-line tumor treatment and to devise an optimal therapeutic approach to overcome a relapse following chemotherapy, there are still many tumor indications with a high medical need and only a limited number of treatment options remain available to patients with relapsed disease and the majority of such patients eventually die. DC vaccines may be a novel therapeutic option for such tumor patients, which could improve their life expectancy and quality of life due to an expected very moderate side effect compared to e.g. standard chemotherapeutic agents. But the hope pinned on DC vaccines so far has not delivered significant improvements to the patients with only one approved DC vaccine, sipuleucel T, which was able to show some additional benefit, which however was rated not quantifiable by the German Institute for Quality and Efficiency in Health Care (IQWIQ) (Press release "Sipuleucel-T in prostate cancer: indication of added benefit" of the IQWIQ dated 19 March 2015; https://www.iqwig.de/en/press/press-releases/press-releases/sipuleucel-t-in-prostate-cancer-indication-of-added-benefit.6618.html; retrieved 12 December 2017).Chu et al. report a phase I/II randomized trial of dendritic cell vaccination with or without cyclophosphamide for consolidation therapy of advanced ovarian cancer in first or second remission (Cancer Immunol. Immunother. 2012, 61(5):629-641).

Besides the identification of optimized manufacturing protocols for DC vaccines, there is still a need for optimal treatment schedules in combination with standard chemotherapies to establish DC vaccines as an effective treatment option in order to prolong the disease-free interval to the next recurrence and next-line treatment and/or to improve survival rates of patients.

The inventors have now surprisingly identified that a DC vaccine administered in parallel to chemotherapy was more beneficial for the overall survival and progression free survival of cancer patients than standard of care chemotherapy alone.

### Definitions:

"Dendritic cell vaccine" or "DC vaccine" refers to human DCs for therapeutic use that may be administered, being prepared without an antigen source or with an antigen source. Preferably, the DCs have been prepared by loading the DCs with an antigen sourced from tumor associated peptide(s), whole antigens from DNA or RNA, whole antigen-protein, idiotype protein, tumor lysate, whole tumor cells or viral vector-delivered whole antigen and subsequently optionally matured with toll-like receptor agonists. More preferably, the DCs have been loaded with whole tumor cells undergoing immunogenic cell death as described for example in (Fucikova et al. 2014). Further, the loaded DCs may be further matured. Maturation occurs e.g. by culturing the loaded DCs in the presence of maturation factors such as Toll-like receptor agonists (e.g., poly[I:C] or LPS).

"Toll-like receptor agonists" refers to molecules binding toll-like receptors (TLR), e.g. lipopolysaccharides binding to TLR4, double-stranded RNA or polyinosinic:polycytidylic acid (poly[I:C]) binding to TLR3. Further, TLR1, TLR2, TLR5 and TLR8 agonists are suitable for maturation. TLR expression and function in DCs is reviewed by Schreibelt et al. (Schreibelt et al. 2010), see table 1 for monocyte derived DCs (moDC).

"Chemotherapy" is a treatment that uses drugs or agents to stop the growth of cancer cells, either by killing the cells (cytotoxic agents) or by stopping them from dividing (cytostatic agents). Chemotherapy drugs may include alkylating agents or alkylating-like agents such as carboplatin or cisplatin, antimetabolites such as gemcitabine, pemetrexed, methotrexate, antitumor antibiotics such as doxorubicin, topoisomerase inhibitors such as topotecan, irinotecan or etoposide, mitotic inhibitors such as docetaxel, paclitaxel, vinblastine, or vinorelbine. Further examples of chemotherapy drugs are provided by the American Cancer Society and can be found here: https://www.cancer.org/treatment/treatments-and-side-effects/treatment-types/chemotherapy/how-chemotherapy-drugs-work.html ("American Cancer Society - How Chemotherapy Drugs Work' 2016). Such drugs may typically be small molecule drugs (organic compounds of low molecular weight, e.g. < 900 Dalton). Chemotherapy may also include other treatments such as hormones and hormone analogous, small molecules drugs (e.g. kinase inhibitors, PARP inhibitors), vaccines other than DC vaccines, or biologics (e.g. antibodies), or combinations thereof. Chemotherapy may be given orally, by injection, or infusion, or on the skin, depending on the type and stage of the cancer being treated. It may be given alone or with other treatments, such as surgery, radiation therapy.

In the context of the present invention, the term "chemotherapy" in a preferred embodiment excludes any agents which are used in the context of cancer therapy to reduce the regulatory T cell response. Thus in one embodiment of the present invention, the chemotherapeutic agent is not cyclophosphamide (Berd and Mastrangelo 1987; Fucikova et al. 2017).

The term chemotherapy also covers "neoadjuvant chemotherapy", which is a chemotherapy treatment given as a first step to shrink a tumor before the main treatment, which is usually surgery.

The term chemotherapy also covers "adjuvant chemotherapy", which is a chemotherapy treatment given after the primary treatment (for example surgery or radiation) to treat residual tumor and/or to prevent or treat metastasis. In the context of the present invention, dendritic cell vaccines are considered not to be adjuvant chemotherapy.

Chemotherapy is typically given in cycles: a treatment period is followed by a recovery period, then another treatment period, and so on. A "cycle" is thus one treatment period followed by one recovery period.

Multiple drugs can be given as "combination chemotherapy" to treat the same disease.

"Completion" of chemotherapy is achieved when the last administration of the chemotherapy within the last cycle is completed. For avoidance of doubt, the last cycle may be the last scheduled cycle or if chemotherapy is "terminated" (e.g. prior to completion due to undesired side effects or non-response to the treatment), it is the last administered cycle.

"Maintenance treatment" is a treatment following the initial chemotherapy or starting within the initial chemotherapy after complete remission or partial response (Sakarya 2016; Ellis 2016). It is given to help keeping cancer from coming back after it has disappeared or shrunk. It may include treatment with selected drugs with moderate side effects, such as hormones and hormone analogous (e.g. enzalutamide, abiraterone, tamoxifen, LHRH agonists and antagonists), kinase inhibitors (e.g. erlotinib, afatinib, gefitinib, crizotinib, alectinib, ceritinib, dabrafenib, trametinib and osimertinib), PARP inhibitors (e.g. olaparib, niraparib), vaccines other than DC vaccines, or biologics (e.g. antibodies as for example bevacizumab, pembrolizumab, nivolumab). Thus, the maintenance treatment has the goal of maintaining the complete remission or partial remission state of the patient. Preferably, it may be given for extended periods of time without causing adverse events leading to termination of the treatment. Excluded from maintenance therapy are interferon/hydroxycloroquine (IFN/HCQ) treatments. Preferably, the initial chemotherapy described above is an adjuvant chemotherapy after which the maintenance treatment is following.

Administration of a DC vaccine may be started with the chemotherapy or while the chemotherapy is ongoing and therefore the DC vaccine therapy is given in "parallel" to chemotherapy. The "parallel" administration comprises that either the DC vaccine therapy and the chemotherapy are both completed or terminated together, or that the DC vaccine therapy continues once the chemotherapy is completed or terminated. The term "parallel" also includes that the DC vaccine can be administered at any stage of an ongoing chemotherapy, e.g. during any cycle of the chemotherapy. Within each cycle, the DC vaccine may be administered during the treatment period and/or the recovery period. "Parallel" administration of chemotherapy and DC vaccine thus implies that chemotherapy overlaps for at least some time with DC vaccine treatment.

A patient will "respond" to chemotherapy if the treatment is effective, i.e. the tumor does not further grow and/or metastasize or the tumor is decreasing in size.

A patient will be "refractory" to chemotherapy if he or she does not respond to the treatment from the beginning or during the course of the chemotherapy, meaning that the cancer is or becomes resistant to the chemotherapeutic drug.

A treated cancer may "relapse" or be "recurrent" when the cancer or signs and symptoms of the cancer return after a period of improvement.

A "biologic" is a substance that is often recombinantly expressed and is used in the prevention, diagnosis or treatment of cancer and other diseases. Biologic agents include antibodies, nucleic acid-based drugs, fusion proteins, hormones or hormone analogs or cytokines. In contrast to most small molecule chemotherapeutic agents that are chemically synthesized and where their structure is known, most biologics are complex mixtures and/or post-translationally modified substances that are not easily identified or characterized. Biological products, including those manufactured by biotechnology, tend to be heat sensitive and susceptible to microbial contamination. In a preferred embodiment of the present invention, a biologic agent is not a virus.

"First-line therapy" is a first treatment given for a disease. It is often part of a standard set of treatments, such as surgery followed by chemotherapy and radiation. When used by itself, first-line therapy is typically the one accepted as the best treatment for a disease. If it does not cure the disease or if it causes severe side effects, other treatment options may be added or used instead. The first-line therapy can also be called induction therapy, primary therapy, or primary treatment.

"First-line chemotherapy" is a therapy with at least one chemotherapeutic agent as part of a first-line therapy. It is understood that the term "first-line chemotherapy" does not comprise a parallel use of a dendritic cell vaccine therapy.

"Second-line chemotherapy" is a chemotherapy that is given when the patient does not respond to the initial treatment (first-line therapy), or if the first-line therapy stops being effective. "Third-line chemotherapy" is the chemotherapy that is given when both initial treatment (first-line therapy) and subsequent treatment (second-line therapy) were not effective or stopped being effective.

"Overall survival" (OS) is the length of time from either the date of diagnosis, date of randomization or the start of treatment for a disease, such as cancer, that patients diagnosed with the disease are still alive. In a clinical trial, measuring the overall survival is one way to measure the efficacy of a new treatment.

"Randomization" into a clinical trial is the process by which subjects are assigned by chance to separate groups that compare different treatments or other interventions. Randomization gives each participant an equal chance of being assigned to any of the groups.

"Overall survival rate" is the percentage of people in a study or treatment group who are still alive for a certain period of time after they were diagnosed with a disease, randomized or started treatment for a disease, such as cancer. The overall survival rate is often stated as a five-year survival rate, which is the percentage of people in a study or treatment group who are alive five years after their diagnosis or the start of treatment.

"Progression-free survival" (PFS) is the length of time during and after the treatment of a disease, such as cancer, that a patient lives with the disease but it does not get worse. In a clinical trial, measuring the progression-free survival is one way to see how well a new treatment works.

A patient is "cured" from cancer when the patient is in complete remission and all signs and symptoms of cancer have disappeared. In a preferred embodiment, a patient is considered as "cured", if he/she remains in complete remission for 5 years or more. Thus, a therapy "failed to cure" a patient, if for example the patient is not in complete remission, if the patient relapsed or if the cancer is refractory.

"Standard of care" (SOC) is a treatment that is accepted by medical experts as a proper treatment for a certain type of disease and that is widely used by healthcare professionals. It can also be called best practice, standard medical care, and standard therapy.

A "serious adverse event" (SEA) is any serious undesirable experience associated with the use of a medical product in a patient. The event is serious when the patient outcome is life-threatening, death, hospitalization/prolongation of hospitalization, congenial anomaly, persistent or significant disability/incapacity requiring intervention to prevent permanent impairment/damage or is an important medical event (e.g. serious problems with breathing blood disorders, seizures/convulsions, drug dependence).

The term "dose" means a measured, defined amount of a drug or DC vaccine administered in a given time, e.g. a daily dose of x cells.

The term "tumor associated peptide(s)" means a peptide that may be associated with a tumor. The presence of the peptide may be highly correlated with the presence of certain tumor cells. They may be highly specific for a certain tumor but the tumor associated peptides may also be related to different tumors. Further, the peptides may also be present not only in tumor cells but also in normal cells, but the tumor associated peptide are then expressed in higher amounts in tumor cells. The tumor associated peptides may also comprise only a partial sequences of a larger tumor associated protein, i.e. a "whole" tumor associated protein. Tumor associated peptides may be isolated from respective tumor cells or they may be produced as recombinant peptides.

The term "whole antigens from DNA or RNA" refers to the case wherein the dendritic cell is loaded with DNA or RNA that encodes the sequence of a whole antigen.

The term "whole antigen-protein" means that the antigen comprises the full-length sequence of a protein of interest as the antigen and not only peptide-epitopes derived from the full-length protein.

The term "idiotype protein" refers to the idiotype target protein, which is composed of the unique antigenic determinants in the variable regions of e.g. clonal immunoglobulin (Ig) expressed by the tumor cells.

The term "tumor lysate" refers to a composition comprising tumor cells and a lysate buffer, wherein the lysate buffer contains agents that lyse the tumor cells. Intracellular, transmembrane and/or extracellular components of the tumor may thus be present in the tumor lysate and serve as antigens in accordance with the present invention.

The term "whole tumor cells" refers to the use of complete tumor cells that have not been lysed in a lysate buffer.

The term "viral vector-delivered whole antigen" means that the antigen is delivered to the dendritic cell by using a viral vector. For example, the whole antigen may be a whole protein and the viral vector encodes the protein and the protein is thus expressed in the dendritic cell after transfection with the viral vector.

The term "taxane" refers to a compound class of diterpenes that feature a taxadiene core structure. Examples of taxanes include, but are not limited to, paclitaxel, docetaxel and cabazitaxel.

The term "platinum complex" refers to metal complex compound, wherein the platinum is the metal component. The platinum may for example be complexed by oxygen or nitrogen atoms of one or more organic or inorganic compounds to form the platinum complex. Examples of platinum complexes include, but are not limited to, carboplatin and cisplatin.

As used herein, the term "newly diagnosed cancer" as in "newly diagnosed ovarian cancer" means that the cancer patient was never diagnosed with that specific cancer, e.g. ovarian cancer before.

As used herein, the term "advanced ovarian cancer", refers to International Federation of Gynaecology and Obstetrics [FIGO] stages **III B, III** C, and IV ovarian cancer.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

The term "at least one" such as in "at least one chemotherapeutic agent" may thus mean that one or more chemotherapeutic agents are meant. The term "combinations thereof" in the same context refers to a combination comprising more than one chemotherapeutic agents.

Technical terms are used by their common sense. If a specific meaning is conveyed to certain terms, definitions of terms will be given in the following in the context of which the terms are used.

### Description of the invention

The invention relates to a DC vaccine for use in the treatment of ovarian cancer wherein the DC vaccine is administered to a patient in parallel to chemotherapy with at least one chemotherapeutic agent as defined in the claims. The chemotherapeutic agent or agents to be used may be determined by the qualified practitioner, and may be part of the standard of care recommended for the type of cancer to be treated. Depending on the standard of care, the chemotherapeutic agent/s may be administered in one or multiple cycles. The duration, frequency and number of cycles will depend on the cancer to be treated and on the agent/s used. The chemotherapeutic agent/s may all be given on a single day within a cycle, several consecutive days, or continuously to an outpatient or to an inpatient. Treatment could last minutes, hours, or days, depending on the specific protocol. Chemotherapy may repeat weekly, bi-weekly, or monthly. Usually, a cycle is defined in monthly intervals. For example, two bi-weekly chemotherapy sessions followed by a recovery period may be classified as one cycle. In most cases, the total number of cycles - or the length of chemotherapy from start to finish - has been determined by research and clinical trials. As long as there are detectable signs of cancer, the length of chemotherapy treatment will depend upon the response of the cancer to therapy. If the cancer disappears completely (complete response), chemotherapy may continue for 1-2 cycles beyond this observation to maximize the chance of having attacked all microscopic, i.e. undetectable, tumors. If the cancer shrinks but does not disappear (partial response), chemotherapy may continue as long as it is tolerated and the cancer does not grow (progression).

If the cancer continues to grow during chemotherapy, the chemotherapy will be terminated and the patient will be considered as refractory to chemotherapy and such chemotherapy will be considered as completed. Chemotherapy will also be considered as completed when the last administration of chemotherapy within the last cycle is completed.

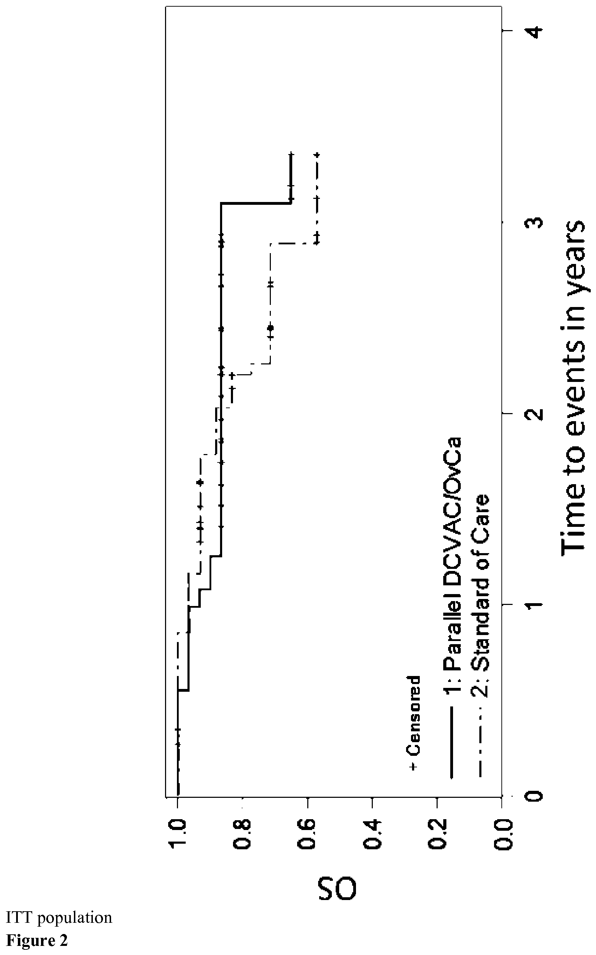

It has surprisingly been found that ovarian cancer patients, receiving DC vaccine in parallel to chemotherapy, had a longer overall survival compared to patients treated with chemotherapy only (see Figures 2, 3, 5, 6 and 8). Even more surprisingly, increase of overall survival was observed when the DC vaccine was combined with different types of chemotherapy combinations (carboplatin/paclitaxel or carboplatin/gemcitabine), when the DC vaccine was used in different treatment settings (combined with first line chemotherapy or second line chemotherapy) and when the DC vaccine was used to treat different types of cancers (e.g. ovarian cancer or lung cancer).

The DC vaccine may be formulated for intravenous, intradermal or subcutaneous administrations, preferably for subcutaneous administration. The DC vaccine can be formulated for example for infusion or bolus injection, and may be administered together with an adjuvant (Elster, Krishnadas, and Lucas 2016). Administration can be systemic, loco-regional or local. When a dose is injected, the dose can be administered in one or multiple injections, preferably in two injections. Preferably, the DC vaccine may be administered to the patient subcutaneously into lymph-node regions close to the region where the cancer to be treated is developing. Various delivery systems are known and can be used to deliver the DC vaccine. The mode of administration may be left to the discretion of the practitioner.

In the context of the present invention, the term chemotherapy is understood to relate to the class of compounds used as cytostatic or cytotoxic agents. In a preferred embodiment, the at least one chemotherapeutic agent is selected from carboplatin, cisplatin, paclitaxel, docetaxel, gemcitabine, pegylated liposomal doxorubicin, etoposide, topotecan, irinotecan, olaparib, rucaparib, trabectedin, niraparib, mitoxantrone, cabazitaxel, vinorelbine, pemetrexed, vinblastine, and albumin-bound paclitaxel. The specific regimen of chemotherapy may be dictated by the established standard of care of the specific cancer to be treated, and may be at the discretion of the practitioner. In a preferred embodiment, cyclophosphamide is not a chemotherapeutic agent. In particular, a low dose cyclophosphamide is not a chemotherapeutic agent according to the present invention; it is used to reduce the function of regulatory T cells (Berd and Mastrangelo 1987; Fucikova et al. 2017) and has been used prior to DC vaccination in the prior art (Dong et al. 2016). Chemotherapy may include drugs prescribed as maintenance therapies or prescribed until progression of the cancer such as hormonal therapies (e.g. enzalutamide, abiraterone, tamoxifen, LHRH agonists and antagonists), targeted therapies (e.g. erlotinib, afatinib, gefitinib, crizotinib, alectinib, ceritinib, dabrafenib, trametinib and osimertinib) and biologics (e.g. antibodies as for example bevacizumab, pembrolizumab, nivolumab). For the avoidance of doubt, chemotherapy in the meaning of this invention does not include drugs prescribed to treat co-morbidities, to avoid or treat side-effects or help the patient to recover from side-effects (e.g. erythropoietin).

The first dose of DC vaccine is administered in parallel to each chemotherapy cycle, and may be further administered after completion/termination of the chemotherapy. The first DC vaccine administration may start with the first cycle of chemotherapy, or with the second chemotherapy cycle, or with the third chemotherapy cycle, or with later chemotherapy cycles. Preferentially, the first DC vaccine dose administration starts after the 2^{nd} cycle of chemotherapy and continues after completion/termination of the chemotherapy. In a preferred embodiment, the patient received at least 6 cycles of chemotherapy.

In a preferred embodiment, DC vaccine is administered to a patient in parallel to chemotherapy with at least one chemotherapeutic agent, where the chemotherapy is first-line chemotherapy. The type of first line treatment will depend on the ovarian cancer to be treated and on the stage of the ovarian cancer (e.g. the TNM staging system - tumor/lymph nodes/metastasis ('National Cancer Institute - Diagnosis and Staging' 2015) or FIGO staging for ovarian cancer ('FIGO ovarian cancer staging' 2014)). For example, in the case of first-line treatment of ovarian cancer, a dendritic cell vaccine may be administered in parallel to a taxane and a platinum complex combination chemotherapy, especially a paclitaxel and carboplatin combination chemotherapy, or a docetaxel and carboplatin combination chemotherapy. Alternatively the chemotherapy combination may be a pegylated liposomal doxorubicin and carboplatin combination. More generally, first-line chemotherapy treatments in use for cancers such as ovarian cancer, prostate cancer and lung cancer, at different stages, where a dendritic cell vaccine may be administered are described at http://www.cancertherapyadvisor.com (CancerTherapyAdvisor 2017b, 2017c, 2017a).

The invention also relates to a DC vaccine treatment administered to a patient in parallel to chemotherapy, where a previous chemotherapy treatment failed to cure the patient from ovarian cancer A previous chemotherapy may have failed to cure the patient because the patient relapsed after completion or termination of the previous chemotherapy or because the patient was refractory to the chemotherapy. Preferably, during a second line chemotherapy setting or later chemotherapy settings, the first DC vaccine dose administration starts after the 2^{nd} cycle of chemotherapy and continues after completion/termination of the chemotherapy.

In another aspect, the DC vaccine for use in the treatment of ovarian cancer may be continued to be administered in combination with a maintenance therapy once the initial chemotherapy is completed. Examples for maintenance therapies for ovarian cancer are treatments with bevacizumab (continuation maintenance after 1^{st} line carboplatin/paclitaxel treatment), olaparib (after 2^{nd} line platinum sensitive treatment and after 3^{rd} line treatment) or niraparib (after 3^{rd} line treatment). Examples for maintenance therapies for non-small cell adenoma carcinoma lung cancer are treatments with bevacizumab after 1^{st} line treatments. In a preferred embodiment, the maintenance therapy comprises the maintenance therapy with bevacizumab, olaparib and/or niraparib.

Different types of DC vaccines are well known in the art and can be divided into different groups according to the method how the DCs are loaded or pulsed with tumor antigens (Turnis and Rooney 2010; Elster, Krishnadas, and Lucas 2016), Accordingly, the DC vaccine for use in the treatment of ovarian cancer administered to a patient in parallel to chemotherapy is a DC vaccine loaded *ex-vivo* with an antigen source which is an antigen source preferably selected from tumor associated peptide(s), whole antigens from DNA or RNA, whole antigen-protein, idiotype protein, tumor lysate, whole tumor cells or viral vector-delivered whole antigen. The DC vaccine may optionally be matured with toll-like receptor (TLR) agonists (e.g. poly[I:C] or LPS). Preferentially, the DC vaccine is matured with the TLR3 agonist poly[I:C]. Tumor lysate may be autologous or allogeneic/heterologous. Likewise, whole tumor cells may be autologous or allogeneic/heterologous. Whole tumor cells and tumor lysate may originate from one or multiple tumor cell lines, and may be selected for a specific presence of antigens matching the patient's tumor to be treated. The source of antigens may be selected and DCs may be loaded by techniques known to one of skill in the art (Turnis and Rooney 2010; Elster, Krishnadas, and Lucas 2016).

In a preferred embodiment of the invention, the antigen source is whole tumor cells and wherein preferably the tumor cells were killed by high hydrostatic pressure (HHP), e.g. as described in WO 2013/004708 and WO 2015/097037, (see examples 1 to 4 of WO 2013/004708 and examples 2 and 3 of WO 2015/097037). In brief, whole tumor cells from cell lines or from the patient are treated by HHP between 200 and 300 MPa for 10 min to 2 hour. Such a treatment will induce a specific type of apoptosis called immunogenic cell death (ICD) in the treated tumor cells which may be characterized by expression of immunogenic molecules on the cell surface such as HSP70, HSP90 and calreticulin and the release of late apoptotic markers HMGB1 and ATP and thus increase the uptake of these cells by DCs, resulting in loaded DCs presenting the multiple tumor antigens. Prior to being loaded on DCs, the apoptotic tumor cells may be cryopreserved.

In a preferred embodiment, the whole tumor cells loaded upon the DC vaccine are allogeneic to the patient. Whereas autologous tumor cells purportedly have a better match with the patient's tumor antigens, in practice it is highly complicated to manufacture a DC vaccine from autologous tumor biopsies. Therefore, it is preferred to use allogeneic tumor cells, e.g. tumor cell lines, which have an overlap of expressed tumor antigens with the typical tumor antigens of the tumor disease to be treated.

In turn, the DCs may be derived from monocytes that are autologous to the patient being treated. As used herein, the term "monocytes" refers to leukocytes circulating in the blood characterized by a bean-shaped nucleus and by the absence of granules. Monocytes can give rise to dendritic cells. The monocytes can be isolated from a patient's blood by any technique known to one of skill in the art, the preferred method being leukapheresis. Leukapheresis allows to collect monocytes that are autologous to the patient being treated, to be used for the preparation of the DC vaccine. Leukapheresis may be performed by any technique known to one of skill in the art.

Typically, dendritic cells are derived from monocytes obtained by leukapheresis prior to chemotherapy, which is combined with the DC vaccination. As many chemotherapies induce neutropenia, a leukapheresis after initiation of chemotherapy may lead to less viable cells and therefore to a lower quantity and/or quality of the DC vaccine.

In another aspect of the invention, 5 to 30 doses of dendritic cell vaccines are administered to the patient and preferentially 8 to 15 doses of dendritic cell vaccines are administered to the patient. In another preferred embodiment, at least 8 doses of dendritic cell vaccines are administered to the patient. During manufacturing, DC vaccines may be aliquoted as multiple aliquots, each aliquot containing the same amount of DC. DC vaccine aliquots can be cryopreserved until use. One or more aliquots may constitute one dose. The number of doses to be administered may be left at the discretion of the treating practitioner. The number of doses administered to the patient may also depend whether the DC vaccine is further administered during maintenance treatment. The number of doses available for the treatment may also depend on the yield of the leukapheresis, but should preferably not be below 15 doses. DC vaccine doses may be administered to a patient in parallel to chemotherapy at 1-week intervals between each dose, at 2-week intervals, at 3-week intervals, at 5-week intervals or at 6-week intervals, and preferably 3-week intervals. The length of the interval may be determined depending on the cancer to be treated, of the stage of the cancer, on the chemotherapy cycle schedule or at the discretion of the practitioner. The length of the interval may also change during the treatment, starting with a short interval for the first doses (e.g. 3-week intervals), followed by a longer interval for the late doses (e.g. 6-week intervals). In a preferred embodiment, the dosing regimen is preferably 5 initial doses administered at 3-week intervals during the chemotherapy treatment period followed by further doses, preferably 5 doses, at 6-week intervals thereafter. This dosing regimen is particularly preferred for the treatment of ovarian cancer and lung cancer.

In another preferred embodiment, the dendritic cell vaccine for use is administered in a dosing schedule comprising 5 doses given at 3-week intervals followed by up to 10 doses at 6-week intervals.

In another embodiment, the invention relates to a DC vaccine treatment of cancer, to be administered to patients in parallel to chemotherapy, where the cancer is ovarian cancer,

In another embodiment, the invention relates to a DC vaccine treatment of cancer, to be administered to patients in parallel to chemotherapy, where the cancer is ovarian cancer, Ovarian cancer includes epithelial ovarian, fallopian tube, or primary peritoneal cancer and may be stage I to IV according to the International Federation of Gynecology and Obstetrics (FIGO).

In another preferred embodiment, the cancer to be treated is ovarian cancer, preferably newly diagnosed epithelial ovarian carcinoma, relapsed platinum-sensitive epithelial ovarian carcinoma, and more preferably newly diagnosed epithelial ovarian carcinoma. In another particularly preferred embodiment, the cancer is recurrent advanced ovarian cancer or relapsed platinum-sensitive epithelial ovarian carcinoma.

In another preferred embodiment, ovarian cancer is advanced ovarian cancer, newly diagnosed ovarian epithelial carcinoma or recurrent ovarian cancer.

In a preferred embodiment, the at least one chemotherapeutic agent is selected from the group consisting of carboplatin, cisplatin, paclitaxel, docetaxel, gemcitabine, pegylated liposomal doxorubicin, etoposide, topotecan, irinotecan, olaparib, rucaparib, trabectedin, niraparib, enzalutamide, abiraterone, mitoxantrone, cabazitaxel, vinorelbine, pemetrexed, methotrexate, vinblastine, albumin-bound paclitaxel, erlotinib, afatinib, gefitinib, crizotinib, alectinib, ceritinib, dabrafenib, trametinib and osimertinib; and biologics or a combination thereof.

In another preferred embodiment, the chemotherapeutic agent is selected from the group consisting of carboplatin, cisplatin, paclitaxel, docetaxel, gemcitabine, pegylated liposomal doxorubicin, etoposide, topotecan, irinotecan, trabectedin, mitoxantrone, cabazitaxel, vinorelbine, pemetrexed, methotrexate, vinblastine, and albumin-bound paclitaxel and combinations thereof.

In a preferred embodiment, the chemotherapy treatment also includes combination treatment of at least two chemotherapeutic agents. In one embodiment, the chemotherapy treatment comprises a combination treatment selected from a taxane and a platinum complex; gemcitabine and a platinum complex; cisplatin and vinorelbine; cisplatin and etoposide; cisplatin and pemetrexed; cisplatin and irinotecan; gemcitabine and docetaxel; carboplatin and etoposide; carboplatin and pemetrexed and gemcitabine and docetaxel. It is particularly preferred that the combination treatment is selected from the following drug combinations: paclitaxel and carboplatin, paclitaxel and cisplatin, docetaxel and carboplatin, cisplatin and vinorelbine, cisplatin and etoposide, cisplatin and gemcitabine, cisplatin and pemetrexed, cisplatin and irinotecan, gemcitabine and docetaxel, carboplatin and etoposide, carboplatin and gemcitabine, carboplatin and pemetrexed and gemcitabine and docetaxel. Combination treatments may be dictated by the established standard of care of the specific cancer to be treated, institutional guidelines and may be at the discretion of practitioner. In a preferred setting, preferably for first line treatment, preferably for the treatment of ovarian cancer or lung cancer, a DC vaccine may be administered in parallel to chemotherapy, wherein the chemotherapy is a taxane and a platinum complex combination, especially the paclitaxel and carboplatin combination. In another preferred setting, especially for the treatment of recurrent advanced ovarian cancer, a DC vaccine may be administered in parallel to chemotherapy, where the chemotherapy is a platinum complex and gemcitabine combination, and preferably cisplatin and gemcitabine or carboplatin and gemcitabine.

In another preferred embodiment, the chemotherapy treatment for newly diagnosed epithelial ovarian carcinoma is a platinum compound combined with a taxane.

In another particularly preferred embodiment, the chemotherapy treatment for newly diagnosed epithelial ovarian carcinoma is carboplatin combined with paclitaxel.

In another preferred embodiment, the chemotherapy treatment of relapsed platinum-sensitive epithelial ovarian cancer is a platinum based compound combined with gemcitabine.

In another particularly preferred embodiment, the chemotherapy treatment of relapsed platinum-sensitive epithelial ovarian cancer is carboplatin combined with gemcitabine.

In yet another embodiment, leukapheresis is performed on patients with 1^{st} recurrence of ovarian cancer prior to the administration of the first cycle of chemotherapy. Following leukapheresis, the collected autologous monocytes are cultivated in the presence of cytokines to obtain immature DCs. Preferred cytokines are GM-CSF and IL-4. The DC vaccine is obtained by loading the immature DCs with tumor cells undergoing ICD, preferably from HHP treated allogeneic tumor cell lines of the same tumor origin as the one to be treated, and further maturation of the loaded DCs with TLR agonists, preferably poly[I:C] resulting in the DC vaccine, which may be fractioned and stored in individual doses of approximately 1x10⁷ DCs per dose. Second-line chemotherapy can be platinum (carboplatin or cisplatin) combined with either paclitaxel, gemcitabine or PEGylated liposomal doxorubicin. Platinum/paclitaxel and platinum/gemcitabine are administered in 21-day cycles; platinum/PEGylated liposomal doxorubicin in 28-day cycles.

In one embodiment, four to six cycles of chemotherapy are administered to the patient, and the DC vaccine administration may start with the first dosing during the second cycle of chemotherapy. For example, the DC vaccine administration starts at the beginning of the third or fourth week after chemotherapy administration of the 21-day cycles or 28-day cycles, respectively. In one embodiment, one dose of the DC vaccine is administered during each of all subsequent chemotherapy cycles. Once the chemotherapy cycles are completed, up to seven further doses of the DC vaccine may be administered as maintenance therapy. In a preferred embodiment, the DC vaccine is administered as maintenance therapy at 6-week intervals. In addition to the chemotherapy and DC vaccine treatment, established maintenance therapy may be administered to the patient during the treatment, e.g. treatment with bevacizumab or PARP inhibitors.

In another embodiment, leukapheresis is performed on patients with stage IV NSCLC prior to the administration of the first cycle of chemotherapy. Following leukapheresis, the collected autologous monocytes are cultivated in the presence of cytokines to obtain immature DCs. Preferred cytokines are GM-CSF and IL-4. The DC vaccine is obtained by loading the immature DCs with tumor cells undergoing ICD, preferably from HHP treated allogeneic NSCLC tumor cell line(s), and further maturation with TLR agonists, preferably poly[I:C] resulting in the DC vaccine, which may be fractioned and stored in individual doses of approximately 1x10⁷ DCs per dose.

In one embodiment, four to six three-week cycles of chemotherapy (e.g., carboplatin and paclitaxel) are administered to the patients. The first DC vaccine dose administration may occur during the second cycle of chemotherapy. For example, the first DC vaccine dose administration may occur at the beginning of the third week. In one embodiment, the DC vaccine is administered during all the subsequent chemotherapy cycles. Once the chemotherapy cycles are completed, up to eleven further doses of the DC vaccine may be administered as maintenance therapy. In a preferred embodiment, the DC vaccine is administered as maintenance therapy at six-week intervals.

### Description of the drawings

**Figure 1****:**
   Schematic diagram of the clinical study on patients with newly diagnosed ovarian cancer, comparing the standard of care chemotherapy treatment to the DC vaccine treatment administered in parallel to chemotherapy. "DCVAC OvCa" stands for a dendritic cell vaccine wherein dendritic cells have been loaded with ovarian cancer cells undergoing immunogenic cell death and matured by a Toll-like receptor ligand. The treatment period 1 corresponds to the period of chemotherapy, also including DC vaccine administration for treatment Group A. The treatment period 2 corresponds to the period when the DC vaccine was continued to be administered in treatment Group A, after completion of the chemotherapy.
**Figure 2****:**
   Interim analysis of OS in the clinical study for the ITT population with newly diagnosed ovarian cancer. "Standard of care" stands for chemotherapy treatment alone.
**Figure 3****:**
   Interim analysis of OS in the clinical study for the PP population with newly diagnosed ovarian cancer. "Standard of care" stands for chemotherapy treatment alone.
**Figure 4****:**
   Schematic diagram of the clinical study on patients with 1^{st} recurrence of ovarian cancer, comparing the standard of care chemotherapy treatment to the DC vaccine treatment administered in parallel to chemotherapy. "DCVAC OvCa" stands for a dendritic cell vaccine wherein dendritic cells have been loaded with ovarian cancer cells undergoing immunogenic cell death and matured by a Toll-like receptor ligand. A day 1/8 regimen means that gemcitabine is given on day 1 and 8 of each cycle and carboplatin is given on the first day of every cycle. The length of a cycle is 21 days.
**Figure 5****:**
   Interim analysis (A) and final analysis (B) of OS in the clinical study for the ITT population with 1^{st} recurrence of ovarian cancer. "Standard of care" stands for chemotherapy treatment alone.
**Figure 6****:**
   Interim analysis (A) and final analysis (B) of OS in the clinical study for the PP population with 1^{st} recurrence of ovarian cancer. "Standard of care" stands for chemotherapy treatment alone.
**Figure 7****:**
   Schematic diagram of the clinical trial, stage IV non-small cell lung cancer, comparing the standard of care chemotherapy treatment to the DC vaccine treatment administered in parallel to chemotherapy. "DCVAC LuCa" stands for a dendritic cell vaccine wherein dendritic cells have been loaded with lung cancer cells undergoing immunogenic cell death and matured by a Toll-like receptor ligand. The "treatment period" is the total period of treatment, including the period with the chemotherapy and DC vaccine combination and the period DC vaccine alone after completion of the chemotherapy. The short 3-week cycles correspond to the chemotherapy cycles, including DC vaccine starting during the 2^{nd} chemotherapy cycle. The 11 long cycles correspond to the period when the DC vaccine is given alone after completion of the chemotherapy.
**Figure 8****:**
   Interim analysis of OS in the clinical study for the PP population with stage IV non-small cell lung cancer.
**Figure 9****:**
   Interim analysis of PFS in the clinical study for the PP population with stage IV non-small cell lung cancer.
   "Censored" in Figure 2, 3, 5, 6, 8 and 9 means patients who are still included in the study by the time of reporting. Censored subjects are indicated on the Kaplan-Meier curve as tick marks; these do not terminate the interval.
**Figure 10****:**
   Schedule of DCVAC OvCa administration during the chemotherapy regimen. An ideal situation is shown with chemotherapy cycles strictly 21/28 days. In case they are not 21/28 days, the succeeding cycle of DCVAC OvCa will be calculated from the date of the last clinic visit, where the patient comes to the clinic to receive DCVAC. "IMP" stands for Investigational Medicinal Product and is DCVAC OvCa. The arrows indicate the ideal day of administration and the doted arrows ± 3 days.

### Examples

### Example 1. DC vaccine

The DC vaccine consisted of autologous DCs loaded *ex vivo* with killed ovarian cancer cells and matured by a Toll-like receptor 3 (TLR-3) ligand. DCs were derived from autologous monocytes that were obtained by leukapheresis. Monocytes isolated from the leukapheresis product were cultured in the presence of granulocyte macrophage colony-stimulating factor and interleukin 4 to obtain immature DCs. Immature DCs were loaded with cells of the ovarian cancer cell lines OV-90 and SK-OV-3 (in a ratio of 2:1). Before being added to the DC culture, OV-90 and SK-OV-3 cells were treated with high hydrostatic pressure (HHP) (as described in WO 2013/004708, examples 1-4), which induces immunogenic cell death (Fucikova et al. 2014). The tumor cell-loaded DCs were matured by polyinosinic:polycytidylic acid (poly[I:C]), a TLR-3 ligand.

The final product was cryopreserved in doses of approximately 1x10⁷ DCs per vial in 1 mL of CryoStor CS10 freezing medium containing 10% dimethyl sulfoxide.

DC vaccine aliquots were transported to the study sites on dry ice at a temperature below -50°C. Each DC vaccine dose was then thawed and diluted in saline to a final volume of 5 mL. The diluted dose was administered to the patient subcutaneously in two applications: one into the inguinal area and one into the contralateral axillary area (2.5 mL to each of the application sites).

### Example 2. Systemic standard therapy in newly diagnosed ovarian cancer patients

The European Society of Medical Oncology (ESMO) established guidelines (Ledermann et al. 2013) of treatment options for patients with newly diagnosed ovarian cancer.
- Stage I
   ∘ Surgery is the initial method aiming at resection of the tumour and adequate staging of the disease.
   ∘ Adjuvant chemotherapy (after completion of surgery):
      ▪ Platinum-based chemotherapy with carboplatin
      ▪ Combination of carboplatin with paclitaxel
- Stage II-IV
   ∘ Surgery should be focused on complete cytoreduction of all macroscopic disease. Benefit of systematic pelvic and para-aortic lymphadenectomy is considered questionable at this stage.
   ∘ Chemotherapy in all patients (after or before surgery)
      ▪ Paclitaxel with carboplatin combination has been the standard of care for over 15 years. Carboplatin can be replaced by cisplatin; however, cisplatin is more toxic and its administration is less convenient.
      ▪ In case of paclitaxel toxicity, docetaxel in combination with carboplatin or pegylated liposomal doxorubicin with carboplatin are considered acceptable options.
      ▪ Alternative schemes of paclitaxel and carboplatin administration, such as intraperitoneal administration or dense-dose regimens, have also been considered, however they are not supported as a standard of care due to the lack of confirmatory data.
   ∘ Targeted therapy: Bevacizumab (biologic, anti-VGEF-A antibody) as an add-on therapy to first-line chemotherapy is indicated for the front-line treatment of adult patients with advanced (International Federation of Gynaecology and Obstetrics [FIGO] stages III B, III C, and IV) epithelial ovarian, fallopian tube, or primary peritoneal cancer, followed by continued use of bevacizumab as a single agent until disease progression or for a maximum of 15 months or until unacceptable toxicity, whichever occurs earlier.

Surgery followed by first-line platinum-based chemotherapy is the standard treatment in most patients, improving their PFS and OS. Nevertheless, despite the initial response, approximately 70% of patients with advanced disease relapse and die within 5 years (Ledermann et al. 2013).

### Example 3. Standard therapy after first recurrence of platinum-sensitive ovarian cancer

The European Society of Medical Oncology (ESMO) established guidelines (Ledermann et al. 2013) of treatment options for therapy after first recurrence of platinum-sensitive ovarian cancer. It also included recently authorized maintenance treatments.
- Second-line therapy: taxane in combination with a platinum compound (platinum doublet) is considered valuable, with other agent combinations tested in clinical trials, including gemcitabine, trabectedin and pegylated liposomal doxorubicin. The choice should be based on the toxicity profile and convenience of administration.
- Targeted therapy: bevacizumab in combination with carboplatin and gemcitabine has been approved and is recommended in platinum-sensitive relapsed disease in patients not previously treated with bevacizumab.
- Maintenance treatments after second-line chemotherapy: olaparib, a PARP inhibitor, is indicated as monotherapy for the maintenance treatment of adult patients with platinum sensitive relapsed BRCA-mutated (germline and/or somatic) high-grade serous epithelial ovarian, fallopian tube, or primary peritoneal cancer who are in response (complete response or partial response) to platinum-based chemotherapy. Niraparib, another PRAP inhibitor, is indicated for the maintenance treatment of adult patients with recurrent epithelial ovarian, fallopian tube, or primary peritoneal cancer who are in complete or partial response to platinum-based chemotherapy.

Despite great efforts to devise an optimal therapeutic approach to overcome the relapse following chemotherapy, only a limited number of treatment options remain available to patients with relapsed disease and the majority of such patients eventually die. Patients with disease recurrence are in high need of novel therapeutic options, such as a DC vaccine, which could improve their life expectancy and quality of life.

### Example 4. Clinical data in newly diagnosed patients with advanced ovarian cancer

The study was conducted as an open label, multicenter phase II clinical trial in women with newly diagnosed epithelial ovarian carcinoma who have undergone debulking surgery. The aim of this study was to evaluate the efficacy and safety of the DC vaccine administered in parallel to standard of care chemotherapy with carboplatin and paclitaxel compared to chemotherapy alone.

A total of 65 patients were centrally randomized in a ratio of 1:1 to treatment groups
- A (34 patients) to receive the DC vaccine in parallel with the standard of care chemotherapy,
- B (31 patients) to receive the standard of care chemotherapy alone.

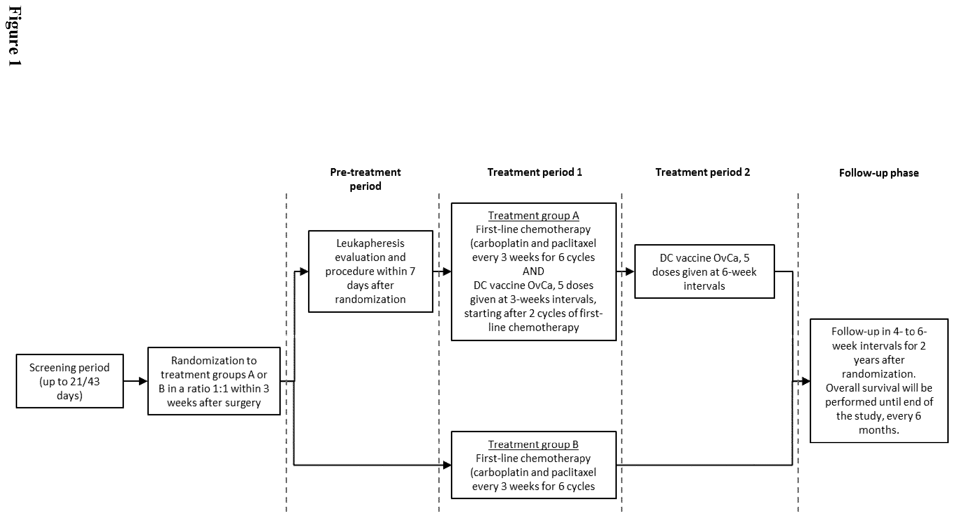

The DC vaccine was administered subcutaneously (SC) to patients in treatment group A in up to 10 doses. The planned number of chemotherapy cycles was 6 in all treatment groups (Figure 1).

The intent-to-treat (ITT) population included all randomized patients regardless of whether they received treatment or not; however, patients randomized to treatment group A had to receive at least 1 dose of DC vaccine to be included in the ITT population (ITT: 31 patients in treatment group A (parallel DC vaccine) and 31 patients in treatment group B (standard of care).

The per-protocol (PP) population included all randomized patients who received at least 3 cycles of standard of care chemotherapy and, for treatment groups A, at least 8 doses of DC vaccine, did not violate any inclusion criteria, and did not have any major protocol deviation (PP: 29 patients in treatment group A (parallel DC vaccine) and 30 patients in treatment group B (standard of care).

The two treatment groups were balanced with regard to demographics and disease characteristics (Table 1). Exposure to chemotherapy and DC vaccine is summarized in Table 2.

**Table 1: Baseline patient demographics and disease characteristics, ITT population**

| | | A | B |
|---|---|---|---|
| | | N=31 | N=31 |
| Age: mean ± SD, median (min - max) | | 58.8 ± 12.1,62 (24-73) | 61.1 ± 7.5, 62 (46-74) |
| Race white: n (%) | | 31 (100%) | 31 (100%) |

| Type of epithelial cells | | | |
|---|---|---|---|
| | Endometrioid, n (%) | 2 (6.45%) | 1 (3.23%) |
| | Mucinous, n (%) | 0 (0.00%) | 0 (0.00%) |
| | Serous, n (%) | 29 (93.55%) | 30 (96.77%) |

| Time since diagnosis*, days | | | |
|---|---|---|---|
| | Mean ± SD, median (min - max) | 26.4 ± 17.9, 21 (13-104) | 25.2 ± 17.9, 21 (11-112) |
| ECOG: mean ± SD, median (min - max) | | 0.581 ± 0.720, 0 (0-2) | 0.452 ± 0.675, 0 (0-2) |

| | | | |
|---|---|---|---|
| * to onset of 1^{st} chemotherapy | | | |

**Table 2: Treatment in SOV01 groups, ITT population**

| | **A** | **B** |
|---|---|---|
| | **N=31** | **N=31** |
| No. of 1^{st}-line chemotherapy cycles: mean ± SD, median (min - max) | 5.90 ± 0.40, 6 (4-6) | 5.68 ± 1.14, 6 (0-6) |
| No. of 1^{st} -line chemotherapy non-responders: n (%) | 1 (3.23%) | 0 (0.00%) |
| No. of DC vaccine doses: mean ± SD, median (min - max) | 9.61 ± 1.43, 10 (3-10) | Not applicable |
| No. of patients with DC vaccine continued beyond progression and administered together with 2^{nd}-line chemotherapy: n (%) | 3 (9.68%) | Not applicable |
| Types of 2^{nd}-line chemotherapy started before completion of DC vaccine ^{a} | 1 patient: doxorubicin & pegylated liposomal doxorubicin | Not applicable |
| | 1 patient: doxorubicin & gemcitabine | |
| | 1 patient: topotecan | |

| | | |
|---|---|---|
| a) The number provided to each second line therapy listed shows the number of patients with the particular treatment. | | |

Results were calculated either by using as beginning of the trial period the time of first treatment or the time of randomization. Two pre-planned per protocol interim analyses of PFS and OS were performed first at 6 months and second at 12 months after the last dose of standard of care chemotherapy of the last enrolled patient. The results from the two analyses were consistent. At the time of the second interim analysis, 17 progression events were reported (PFS data maturity 27.42%) in the treatment group A, and 13 patients died (OS data maturity 20.97% when calculated from the time of treatment, and 19.35% when calculated from the time of randomization). The results from the second interim analysis are presented below. The median duration of patient follow-up at the time of the second interim analysis was 22.9 months (686 days) from the time of treatment and 22.8 months (694 days) from the time of randomization.

### Efficacy in the ITT population

A clear trend towards improved OS was shown in treatment group A (parallel DC vaccine) when compared to treatment group B (standard of care) (hazard ratio (HR) = 0.64, p = 0.4513 from the time of treatment, p = 0.4460 from the time of randomization; Figure 2). Although significance may not have been reached, this is likely due to the relatively small size of the study. Clearly, after two years fewer fatalities occurred in the parallel treatment group A than in the standard of care treatment group B (see Figure 2).

### Efficacy in the PP population

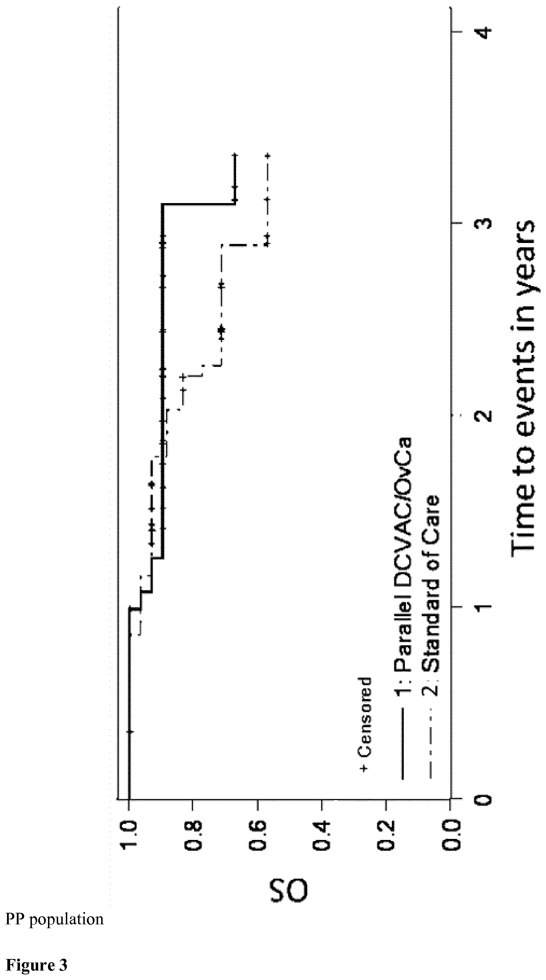

The same trends was observed for the PP population, i.e. after two years a clear trend towards improved OS was shown in treatment group A (parallel DC vaccine) when compared to treatment group B (standard of care) (HR = 0.51, p = 0.2777 from the time of treatment, p = 0.2735 from the time of randomization; Figure 3). Although significance may not have been reached, this is likely due to the relatively small size of the study. Clearly, fewer fatalities occurred in the parallel treatment group A than in the standard of care treatment group B (see Figure 3).

Benefits of a parallel DC vaccine treatment over standard chemotherapy suggested above for the ITT population are therefore further supported by the data for the PP population.

Overall, this clinical data surprisingly show an advantage for patients randomized to the group receiving the DC vaccine in parallel to first-line chemotherapy (group A), compared to standard of care alone (group B). Surprisingly, it was also found that delivering of multiple doses, preferentially at least 8 doses, to a patient was beneficiary to the patient. This can be inferred by the fact that the HR was lower in the PP population than in the ITT (the requirement for inclusion in the PP population was to receive at least 8 administrations of the DC vaccine and 3 cycles of standard of care chemotherapy).

Patient characteristics were balanced between treatment groups A and B (see Table 1 and Table 2). Therefore, the observed advantage in treatment group A compared to treatment group B is not influenced by any demographic.

### Safety data on the DC vaccine

In treatment group A, 14 SAEs were reported in 8 patients before the start of DC vaccine treatment and 14 SAEs were reported in 9 patients after the start of DC vaccine treatment. In treatment group B, 14 SAEs were reported in 9 patients, although on a shorter reporting time period. The most frequently reported SAEs were neutropenia, anemia, thrombocytopenia, ascites and infected lymphocele.

These safety data indicated that the DC vaccine (including leukapheresis) was well tolerated and that there were no safety-related issues associated with the DC vaccine administration. No potential risks have been detected or confirmed in the clinical trials.

### Example 5. Clinical data in first recurrence of ovarian cancer

### Clinical data

The study was a randomized, open-label, parallel group, multicentre, phase II clinical trial evaluating the effect of addition of a DC vaccine to standard of care chemotherapy (carboplatin and gemcitabine) in women with relapsed platinum-sensitive epithelial ovarian cancer. The aim of this study was to explore the efficacy and safety of a DC vaccine administered in parallel to chemotherapy, as an add-on to standard of care chemotherapy with carboplatin and gemcitabine as compared to chemotherapy alone. An interim analysis was first performed, followed later by the final analysis.

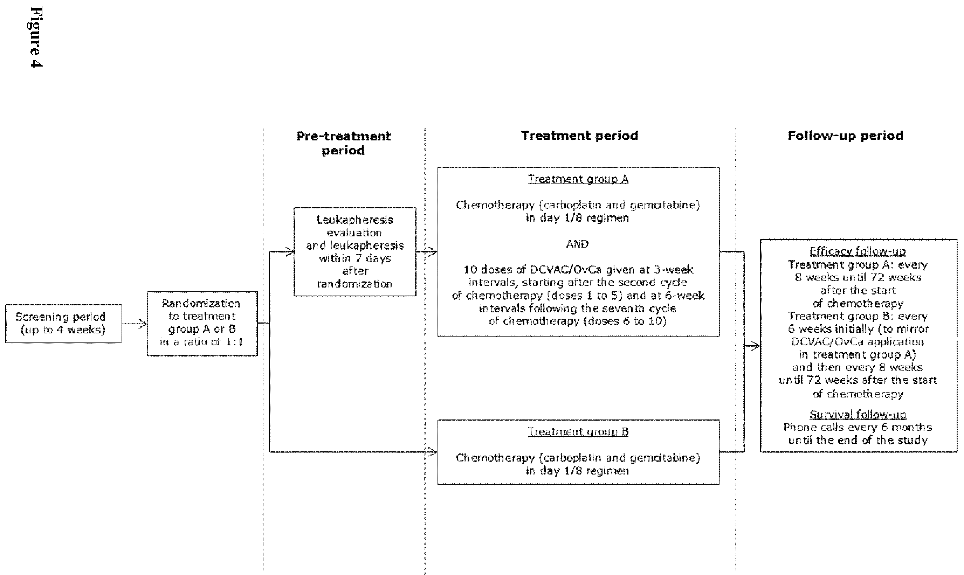

A total of 71 patients were centrally randomized in a ratio of 1:1 to treatment group A (39 patients) to receive the DC vaccine in parallel with standard of care chemotherapy or to treatment group B (32 patients) to receive standard of care chemotherapy alone. The DC vaccine was administered to the patients in treatment group A in up to 10 doses. A total of 6, 8, or 10 cycles of standard of care chemotherapy were to be completed by patients in both treatment groups as per investigators' decision (Figure 4).

The intent-to-treat (ITT) population included all randomized patients regardless of whether they received treatment or not; however, patients randomized to treatment group A had to receive at least 1 dose of DC vaccine to be included into the ITT population (32 patients in treatment group A (parallel DC vaccine) and 32 patients in treatment group B (standard of care).

The per-protocol (PP) population included all randomized patients who received at least 3 cycles of standard of care chemotherapy and, for treatment group A, at least 8 doses of DC vaccine, did not violate any inclusion criteria, and did not have any major protocol deviation (31 patients in treatment group A (parallel DC vaccine) and 27 patients in treatment group B (standard of care).

The two treatment groups were balanced with regard to demographics and oncology history (Table 3). Exposure to chemotherapy and DC vaccine is summarised in Table 4.

**Table 3: Baseline patient demographics and disease characteristics, ITT population**

| | | **Treatment group A** | **Treatment group B** |
|---|---|---|---|
| | | **N=32** | **N=32** |
| Age: n; median years (min, max) | | 32; 58.5 (42, 80) | 32; 60.5 (43, 74) |
| Race white: n (%) | | 32 (100%) | 32 (100%) |
| Type of epithelial cells | | | |
| | Endometrioid, n (%) | 2 (6.3%) | 2 (6.3%) |
| | Mucinous, n (%) | - | 1 (3.1%) |
| | Serous, n (%) | 30 (93.8%) | 29 (90.6%) |
| FIGO III ovarian cancer stage (at diagnosis) n (%) | | 32 (100%) | 32 (100%) |

| For interim analysis: Time since diagnosis to start of treatment, days | | | |
|---|---|---|---|
| | n | 23 | 23 |
| | Mean | 845.9 | 820.0 |
| | Standard deviation | 372.82 | 380.49 |
| | Median | 786.0 | 678.0 |
| | Q1 - Q3 | 643 - 994 | 540 - 980 |
| | Min - max | 364 - 1917 | 388 - 1697 |

| For final analysis: Time since diagnosis to randomization, days | | | |
|---|---|---|---|
| | n | 23 | 23 |
| | Mean | 835.5 | 814.6 |
| | Standard deviation | 372.70 | 378.74 |
| | Median | 771.0 | 672.0 |
| | Q1 - Q3 | 629.0 - 978.0 | 538.0 - 974.0 |
| | Min - max | 352 - 1907 | 383 - 1692 |

**Table 4: Overview of treatments in the study, ITT population**

| | **Treatment group A** | **Treatment group B** |
|---|---|---|
| | **N=32** | **N=32** |
| No. of 2^{nd}-line chemotherapy cycles: mean ± SD, median (min - max) | 6.84 ± 2.52, 6 (1-10) | 5.63 ± 3.01, 6 (0-10) |
| No. of 2^{nd}-line chemotherapy non-responders: n (%) | 1 (3.13%) | 0 (0.00%) |
| No. of DC vaccine doses: mean ± SD, median (min - max) | 9.75 ± 0.80, 10 (6-10) | Not applicable |
| No. of patients with DC vaccine despite further progression, together with third-line chemotherapy: n (%) | 7 (21.88%) | Not applicable |
| Types of 3^{rd}-line chemotherapy while on DC vaccine ^{a} | 2 patients: gemcitabine & cisplatin | Not applicable |
| | 2 patients: paclitaxel & cisplatin | |
| | 1 patient: gemcitabine & carboplatin, then doxorubicin | |
| | 1 patient: gemcitabine & cisplatin, then doxorubicin | |
| | 1 patient: doxorubicin | |

| | | |
|---|---|---|
| *a) The number of patients treated with the particular next-line chemotherapy is indicated* | | |

### Analysis of OS

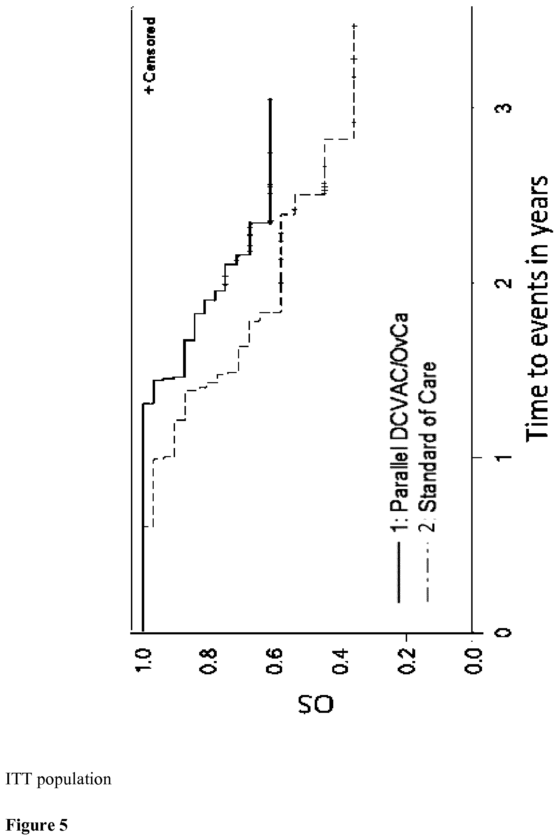

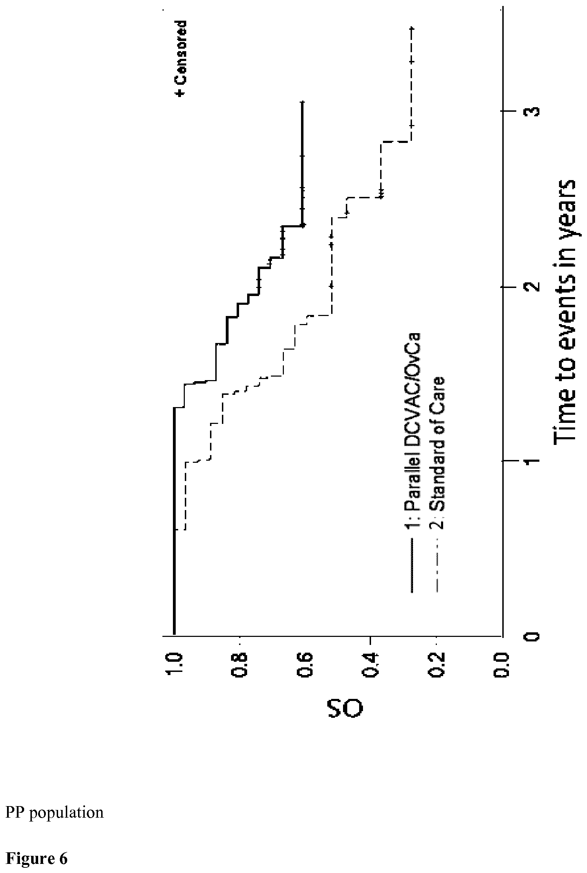

The difference in OS in favour of treatment group A was close to statistical significance in the ITT population (HR = 0.620, p = 0.1565, Table 5, Figure 5A for the interim analysis; HR = 0.38, p = 0.0032, Table 5, Figure 5B for the final analysis) as well as in the PP population (HR = 0.521, p = 0.0662, Table 5, Figure 6A for the interim analysis; HR = 0.38, p = 0.0032, Table 5, Figure 6B for the final analysis) meaning that the risk of death was reduced by 38% in the ITT population and by 47.9% in the PP population, compared to standard of care. Statistical significance has not been reached, likely due to the relatively small size of the study.

**Table 5: Benefit of DCVAC/OvCa on patient survival (data from the ongoing analysis of OS in the study at the time of interim analysis; data from the final analysis)**

| **Interim analysis** | **Treatment group A (parallel DC vaccine) over treatment group B (standard of care)** | | |
|---|---|---|---|
| | **HR** | **p-value** | **Benefit (assessed by medians in months)** |
| **OS (ITT population)** | 0.620 | 0.1565 | Medians 42.9 (extrapolation, not reached) vs 30.0 → expected benefit of OS prolongation by 12.9 months |
| **OS (PP population)** | 0.521 | 0.0662 | Medians 42.2 (extrapolation, not reached) vs 28.6 → expected benefit of OS prolongation by 13.6 months |

| **Final analysis** | **Treatment group A (parallel DC vaccine) over treatment group B (standard of care)** | | |
|---|---|---|---|
| | **HR** | **p-value** | **Benefit (assessed by medians in months)** |
| **OS (ITT population)** | 0.38 | 0.0032 | Medians: 1081 days vs 674 days → OS prolongation by 407 days (= 13.4 months) |
| **OS (PP population)** | 0.38 | 0.0032 | Medians: 1081 days vs 674 days → OS prolongation by 407 days (= 13.4 months) |

### Summary of efficacy data from the study (interim analysis)

The results of the ongoing analysis of OS were close to statistical significance, with signals of a major positive impact on patient survival when DC vaccine was administered in parallel to standard second-line chemotherapy. The expected survival benefit was assessed from extrapolated median survival data. The analysis showed an estimated survival benefit of 12.9 months in patients form the ITT population and 13.6 month in patients from the PP population treated with the DC vaccine added in parallel to standard chemotherapy.

### Summary of efficacy data from the study (final analysis)

The results of the final analysis of OS are statistically significant, with signals of a major positive impact on patient survival when DC vaccine was administered in parallel to standard second-line chemotherapy. The survival benefit was assessed from median survival data. The analysis showed a survival benefit of 13.4 months in patients form the ITT population and 13.4 month in patients from the PP population treated with the DC vaccine added in parallel to standard chemotherapy.

### Example 6. Advantage of a DC vaccine over existing medical practice in a first recurrence setting of ovarian cancer

Platinum combination chemotherapy is currently the treatment of choice for patients with platinum-sensitive ovarian cancer; the selection between platinum-based combinations should be based on the toxicity profile and convenience of administration. Bevacizumab in combination with carboplatin and gemcitabine has been approved and is recommended in platinum-sensitive relapsed disease in patients not previously treated with bevacizumab.

Surprisingly, in the present study, a survival benefit for patients receiving a DC vaccine in parallel with carboplatin and gemcitabine was shown as compared to patients treated only with carboplatin and gemcitabine. This survival benefit was higher than that of the approved treatment of bevacizumab added to carboplatin and gemcitabine or bevacizumab added to carboplatin and paclitaxel (DC vaccine + carboplatin and gemcitabine, ITT population: HR = 0.620, p = 0.1565; bevacizumab + carboplatin and gemcitabine: HR = 0.952, p = 0.6479; bevacizumab + carboplatin and paclitaxel: HR = 0.823, p = 0.0447; Table 6), meaning that the risk of death was reduced by 38% for patients treated with the DC vaccine in combination with carboplatin and gemcitabine when compared with patients treated with carboplatin and gemcitabine only.

**Table 6: Comparison overview of concurrent DCVAC/OvCa and concurrent bevacizumab in patients with platinum-sensitive recurrent ovarian cancer**

| **Combination regimen** | **Administration** | | **Efficacy Interim analysis** | | **Efficacy Final Analysis** | | **Safety** |
|---|---|---|---|---|---|---|---|
| | **Dose and schedule** | **Duration of treatment** | **PFS (HR, p)** | **OS (HR, p)** | **PFS (HR, p)** | **OS (HR, p)** | |
| | AUC4-AUC5 IV q3w | | Primary analysis^{a} 0.74 0.2897 | Ongoing OS analysis^{b} ***PP population** 0.521,* 0.0662 Medians 42.2 months (extrapolation, not reached) vs 28.6 months → expected benefit of **OS prolongation by 13.6 months *ITT population*** 0.620, 0.1565 Medians 42.9 m (extrapolation, not reached) vs 30.0 months => expected benefit of **OS prolongation by 12.9 months** | ***PP population*** 0.71, 0.2224 | ***PP population*** 0.38, 0.0032 Medians 1081 days vs 674 days → OS **prolongation by 13.4 months** | No potential risks have been detected or confirmed in clinical trials with DC vaccine; no added toxicity to chemotherapy |
| Carboplatin | | 6, 8, or 10 cycles | | | ***ITT population*** 0.73, 0.2736 | | |
| Gemcitabine | 1000 mg/m² IV q3w (day 1 and day 8) | 6, 8, or 10 cycles | | | | ***ITT population*** 0.38, 0.0032 Medians 1081 days vs 624 days → **OS prolongation** by **13.4 months** | |
| DC vaccine | Ca 1x10⁷ DCs as two 2.5 mL injections SC q3w + q6w | 5 + 5 cycles | | | | | |
| | | | | | | | |
| | | | | | | | |
| Carboplatin | AUC4 IV q3w | 6 to 10 cycles | 0.48^{c} | 0.952^{c} | | | Patients treated with bevacizumab may be at an increased risk for the development of^{d}: |
| Gemcitabine | 1000 mg/m² IV q3w (day 1 and day 8) | 6 to 10 cycles | | | | | |
| | | | | | | | - gastrointestinal perforation and fistulae |
| | | | <0.0001 | 0.6479 | | | |
| Bevacizumab | 15 mg/kg IV q3w | Until disease progression or unacceptable toxicity | | | | | - wound healing complications |
| | | | | | | | - hypertension |
| | | | | | | | - posterior reversible |
| Carboplatin | AUC5 IV q3w | 6 to 8 cycles | 0.613^{c} | 0.823^{c} | | | encephalopathy syndrome |
| | | | | | | | - proteinuria |
| | | | | | | | - arterial thromboembolis m |
| Paclitaxel | 175 mg/m² IV q3w | 6 to 8 cycles | <0.0001 | 0.0447 | | | |
| | | | | | | | - venous thromboembolis |
| Bevacizumab | 15 mg/kg IV q3w | Until disease progression or | | | | | m - haemorrhage |
| | | unacceptable toxicity | | | | | - pulmonary haemorrhage/ haemoptysis |
| | | | | | | | - congestive heart failure |
| | | | | | | | - neutropenia and infections |
| | | | | | | | - hypersensitivity reactions/infusio n reactions |
| | | | | | | | - osteonecrosis of the jaw |

| | | | | | | | |
|---|---|---|---|---|---|---|---|
| a) Primary analysis of data from the study, ITT population b) Analysis of OS c) Avastin Summary of Product Characteristics ('Avastin Summary of Product Characteristics') AUC, area under the curve; IV, intravenous; DCs, dendritic cells; SC, subcutaneous; q3w, every 3 weeks; q6w, every 6 weeks; PFS, progression-free survival; OS, overall survival; HR, hazard ratio; p, p-value | | | | | | | |

### Example 7. Clinical data in metastatic stage IV non-small cell lung cancer

### Clinical data

The study was conducted as an open label, multicenter phase II clinical trial in patients with metastatic stage IV non-small cell lung cancer (NSCLC). The purpose of the study is to compare efficacy of DCVAC/LuCa + chemotherapy (carboplatin and paclitaxel) vs. carboplatin and paclitaxel alone in patients with stage IV NSCLC, as measured by OS and progression free survival (PFS). PFS was measured based on radiological assessments according to the RECIST (Response Evaluation Criteria in Solid Tumors) guidelines (version 1.1) (Eisenhauer et al. 2009).

A total of 62 patients were centrally randomized in a ratio of 1:1 to treatment group
- A (33 patients) to receive the DC vaccine in parallel with standard of care chemotherapy,
- B (29 patients) to receive standard of care chemotherapy alone.

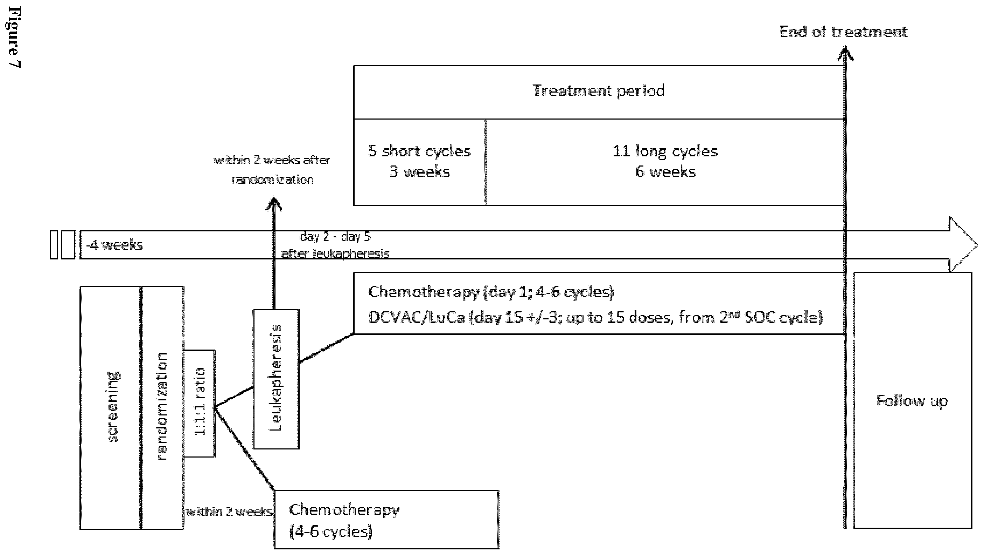

The DC vaccine was administered subcutaneously (SC) to patients in treatment group A in up to 15 doses. The planned number of chemotherapy cycles was 4-6 in all treatment groups (Figure 7).

The per protocol (PP) population excluded patients with DC vaccine production failure and protocol violations; furthermore, patients randomized to treatment group A had to receive at least 1 dose of DC vaccine to be included in the PP population.

The two treatment groups were balanced with regard to demographics and disease characteristics.

### Summary of efficacy data from the study

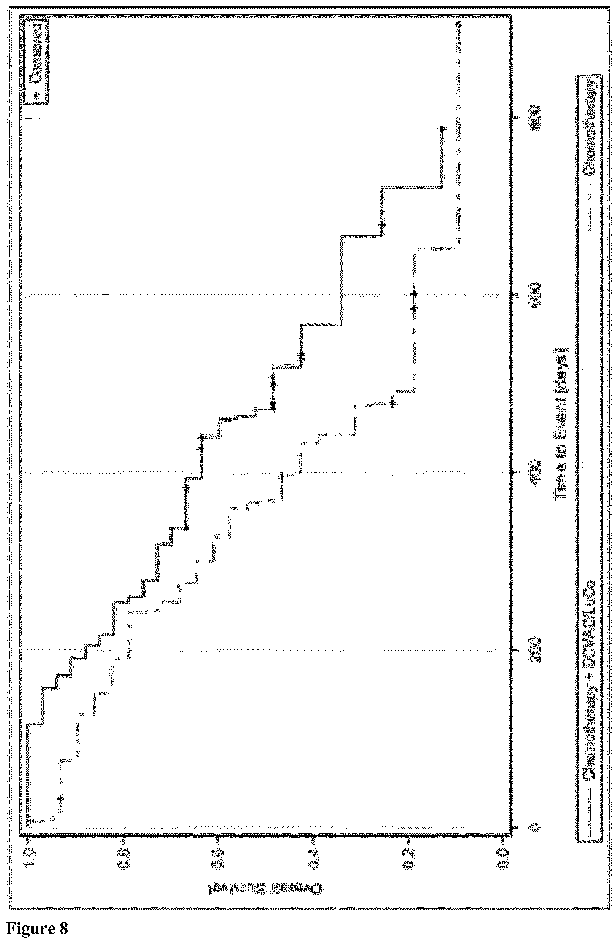

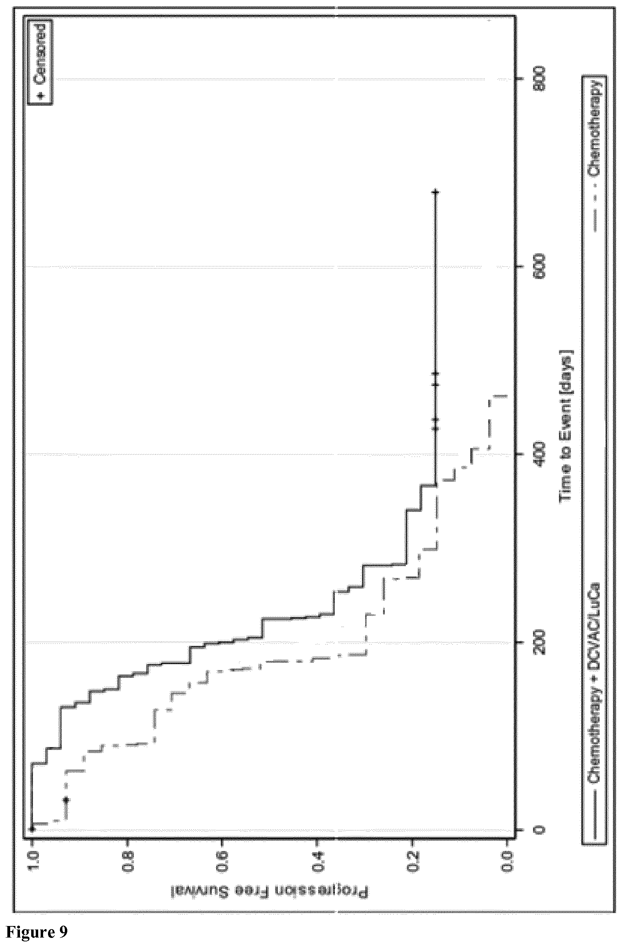

By the time of analysis, the results of the study showed that when the DC vaccine was administered in parallel to standard second-line chemotherapy, OS was improved for patients treated with the DC vaccine in parallel to chemotherapy, the Kaplan Meier estimates for OS showing a positive trend for the DC vaccine in parallel to chemotherapy treatment arm (see Figure 8). Furthermore, the analysis showed that the lung cancer disease eventually progressed for all patients treated with chemotherapy only, while 5 patients (corresponding to 20% of the PP population) treated with the DC vaccine in parallel to chemotherapy did not show any progression of the cancer (see Figure 9).

### Example 8. Clinical trial to test the treatment of patients with 1^{st} recurrence of ovarian cancer with DCVAC/OvCa

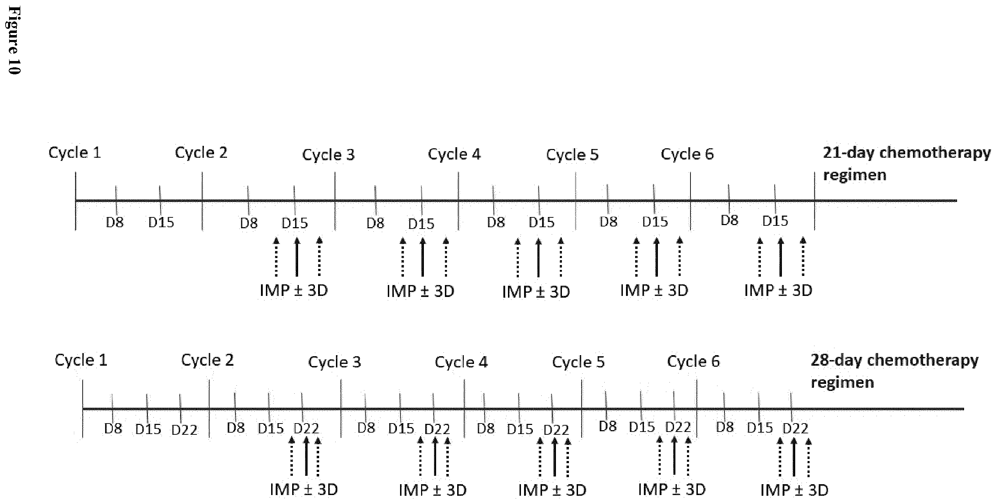

A clinical study is planned as follows. Patients with 1^{st} recurrence of ovarian cancer will be randomized at the start of the treatment at a ratio of 2:1 to either DCVAC/OvCa + second line chemotherapy or second line chemotherapy alone (control group) treatment groups. Leukapheresis will be performed on all patients prior to the administration of the first cycle of chemotherapy. Following leukapheresis, the collected autologous monocytes will be cultivated in the presence of GM-CSF and IL-4 according to Truxova et al (Truxova et al. 2014) to obtain immature DCs. DCVAC/OvCa will be obtained by loading over-night the immature DCs with ovarian cancer tumor cells from the two cell lines SK-OV-3 (obtained from Memorial Sloan-Kettering Cancer Center) and OV-90 (obtained from VAL-CHUM) undergoing ICD due to HHP treatment according to Example 1 and WO 2013/004708, examples 1-4, and further maturation with the TLR agonist poly[I:C]. Upon maturation, the DCs will be aliquoted in cryopreservant in doses of approximately 1x10⁷ DCs per dose and subsequently cryopreserved. Second-line chemotherapy will be platinum (carboplatin or cisplatin) combined with either paclitaxel, gemcitabine or PEGylated liposomal doxorubicin. Platinum/paclitaxel and platinum/gemcitabine will be administered in 21-day cycles, while platinum/PEGylated liposomal doxorubicin will be administered in 28-day cycles, each according to the instructions of the marketing authorization. Four to six cycles of the chemotherapy will be administered to the patient, and the DC vaccine administration will start during the second cycle of chemotherapy. The first DC vaccine administration will occur during the second cycle of chemotherapy at the beginning of the third or fourth week after chemotherapy administration of the 21-day cycles or 28-day cycles, respectively (see Figure 10). DC vaccine will be administered during all the subsequent chemotherapy cycles. Once the chemotherapy cycles will be completed, up to seven further doses of DC vaccine will be administered as maintenance therapy at 6-week intervals. A placebo will be administered to the control group instead of DCVAC/OvCa. In addition to the chemotherapy and DC vaccine treatment, maintenance therapy may be administered to the patient during the treatment. Maintenance therapy will be bevacizumab or PARP inhibitors.

### References

'American Cancer Society - How Chemotherapy Drugs Work'. 2016. American Cancer Society, Accessed 2018/01/05. https:/ /www.cancer.org/treatment/treatments-and-side-effects/treatment-types/chemotherapy/how-chemotherapy-drugs-work.html.
'Avastin Summary of Product Characteristics'. Accessed October 09, 2017. http://www.ema.europa.eu/docs/en_GB/document_library/EPAR - Product_Information/human/000582/WC500029271.pdf.
Banchereau, J., and R. M. Steinman. 1998. 'Dendritic cells and the control of immunity', Nature, 392: 245-52.
Berd, D., and M. J. Mastrangelo. 1987. 'Effect of low dose cyclophosphamide on the immune system of cancer patients: reduction of T-suppressor function without depletion of the CD8+ subset', Cancer Res, 47: 3317-21.
Bloy, N., J. Pol, F. Aranda, A. Eggermont, I. Cremer, W. H. Fridman, J. Fucikova, J. Galon, E. Tartour, R. Spisek, M. V. Dhodapkar, L. Zitvogel, G. Kroemer, and L. Galluzzi. 2014. 'Trial watch: Dendritic cell-based anticancer therapy', Oncoimmunology, 3: e963424.
CancerTherapyAdvisor. 2017a. 'NON-SMALL CELL LUNG CANCER TREATMENT REGIMENS', Accessed 2017/11/24. http://www.cancertherapyadvisor.com/lung-cancer/lung-cancer-nsclc-treatment-regimens/article/527545/.
-. 2017b. 'OVARIAN CANCER TREATMENT REGIMENS', Accessed 2017/11/24. http://www.cancertherapyadvisor.com/gynecologic-cancer/ovarian-cancer-treatment-regimens/article/218127/.
-. 2017c. "PROSTATE CANCER TREATMENT REGIMENS', Accessed 2017/11/24. http://www.cancertherapyadvisor.com/prostate-cancer/prostate-cancer-treatment-regimens/article/218186/?DCMP=OTC-cta_regasset.
Dong, W., R. Wei, H. Shen, Y. Ni, L. Meng, and J. Du. 2016. 'Combination of DC Vaccine and Conventional Chemotherapeutics', Anticancer Agents Med Chem, 16: 558-67.
Eisenhauer, E. A., P. Therasse, J. Bogaerts, L. H. Schwartz, D. Sargent, R. Ford, J. Dancey, S. Arbuck, S. Gwyther, M. Mooney, L. Rubinstein, L. Shankar, L. Dodd, R. Kaplan, D. Lacombe, and J. Verweij. 2009. 'New response evaluation criteria in solid tumours: revised RECIST guideline (version 1.1)', Eur J Cancer, 45: 228-47.
Ellis, P. M. 2016. 'Anti-angiogenesis in Personalized Therapy of Lung Cancer', Adv Exp Med Biol, 893: 91-126.
Elster, J. D., D. K. Krishnadas, and K. G. Lucas. 2016. 'Dendritic cell vaccines: A review of recent developments and their potential pediatric application', Hum Vaccin Immunother, 12: 2232-9.
'FIGO ovarian cancer staging'. 2014. Accessed September 14, 2017. https://www.sgo.org/wp-content/uploads/2012/09/FIGO-Ovarian-Cancer-Staging_1.10.14.pdf.
Fucikova, J., I. Moserova, I. Truxova, I. Hermanova, I. Vancurova, S. Partlova, A. Fialova, L. Sojka, P. F. Cartron, M. Houska, L. Rob, J. Bartunkova, and R. Spisek. 2014. 'High hydrostatic pressure induces immunogenic cell death in human tumor cells', Int J Cancer, 135: 1165-77.
Fucikova, J., M. Podrazil, L. Jarolim, P. Bilkova, M. Hensler, E. Becht, Z. Gasova, J. Klouckova, J. Kayserova, R. Horvath, A. Fialova, K. Vavrova, K. Sochorova, D. Rozkova, R. Spisek, and J. Bartunkova. 2017. 'Phase I/II trial of dendritic cell-based active cellular immunotherapy with DCVAC/PCa in patients with rising PSA after primary prostatectomy or salvage radiotherapy for the treatment of prostate cancer', Cancer Immunol Immunother.
Galluzzi, L., L. Senovilla, E. Vacchelli, A. Eggermont, W. H. Fridman, J. Galon, C. Sautes-Fridman, E. Tartour, L. Zitvogel, and G. Kroemer. 2012. "Trial watch: Dendritic cell-based interventions for cancer therapy', Oncoimmunology, 1: 1111-34.
Garg, A. D., M. Vara Perez, M. Schaaf, P. Agostinis, L. Zitvogel, G. Kroemer, and L. Galluzzi. 2017. "Trial watch: Dendritic cell-based anticancer immunotherapy', Oncoimmunology, 6: e1328341.
Ledermann, J. A., F. A. Raja, C. Fotopoulou, A. Gonzalez-Martin, N. Colombo, C. Sessa, and Esmo Guidelines Working Group. 2013. 'Newly diagnosed and relapsed epithelial ovarian carcinoma: ESMO Clinical Practice Guidelines for diagnosis, treatment and follow-up', Ann Oncol, 24 Suppl 6: vi24-32.
'National Cancer Institute - Diagnosis and Staging'. 2015. National Cancer Institute, Accessed 2017/12/08. https://www.cancer.gov/about-cancer/diagnosis-staging/stagine.
Palucka, K., and J. Banchereau. 2013. 'Dendritic-cell-based therapeutic cancer vaccines', Immunity, 39: 38-48.
Sakarya, D. K.; Yetimalar, M. H. 2016. 'Is there a current change of maintenance treatment in ovarian cancer? An updated review of the literature ', JBUON, 21: 290-300.
Schreibelt, G., J. Tel, K. H. Sliepen, D. Benitez-Ribas, C. G. Figdor, G. J. Adema, and I. J. de Vries. 2010. 'Toll-like receptor expression and function in human dendritic cell subsets: implications for dendritic cell-based anti-cancer immunotherapy', Cancer Immunol Immunother, 59: 1573-82.
Truxova, I., K. Pokoma, K. Kloudova, S. Partlova, R. Spisek, and J. Fucikova. 2014. 'Day 3 Poly (I:C)-activated dendritic cells generated in CellGro for use in cancer immunotherapy trials are fully comparable to standard Day 5 DCs', Immunol Lett, 160: 39-49.
Turnis, M. E., and C. M. Rooney. 2010. 'Enhancement of dendritic cells as vaccines for cancer', Immunotherapy, 2: 847-62.
Vacchelli, E., I. Vitale, A. Eggermont, W. H. Fridman, J. Fucikova, I. Cremer, J. Galon, E. Tartour, L. Zitvogel, G. Kroemer, and L. Galluzzi. 2013. 'Trial watch: Dendritic cell-based interventions for cancer therapy', Oncoimmunology, 2: e25771.
Verma, R., R. E. Foster, K. Horgan, K. Mounsey, H. Nixon, N. Smalle, T. A. Hughes, and C. R. Carter. 2016. 'Lymphocyte depletion and repopulation after chemotherapy for primary breast cancer', Breast Cancer Res, 18: 10.
WO 2013/004708
WO 2015/097037

## Claims

1. A dendritic cell vaccine for use in the treatment of cancer, wherein the dendritic cell vaccine is administered to a patient in parallel to chemotherapy with at least one chemotherapeutic agent, wherein the cancer is ovarian cancer, and wherein the chemotherapeutic agent is selected from carboplatin, cisplatin, paclitaxel, docetaxel, gemcitabine, pegylated liposomal doxorubicin, etoposide, topotecan, irinotecan, olaparib, rucaparib, trabectedin, niraparib, enzalutamide, abiraterone, mitoxantrone, cabazitaxel, vinorelbine, pemetrexed, methotrexate, vinblastine, albumin-bound paclitaxel, erlotinib, afatinib, gefitinib, crizotinib, alectinib, ceritinib, dabrafenib, trametinib and osimertinib; and biologics, or a combination thereof.

2. The dendritic cell vaccine for use of claim 1, wherein the first dose of the dendritic cell vaccine is administered during the chemotherapy period, and optionally the dendritic cell vaccine is further administered after completion of the chemotherapy.

3. The dendritic cell vaccine for use of claim 1 or claim 2, where the chemotherapy is first-line chemotherapy.

4. The dendritic cell vaccine for use of claim 1 or claim 2, wherein a previous chemotherapy treatment failed to cure the patient from cancer.

5. The dendritic cell vaccine for the use of any one of claims 1 to 4, wherein the dendritic cell vaccine is further administered in combination with a maintenance therapy after completion of the chemotherapy.

6. The dendritic cell vaccine for the use according to any one of claims 1 to 5, wherein the dendritic cell vaccine is loaded *ex-vivo* with an antigen source, wherein preferably the antigen source is selected from tumor associated peptide(s), whole antigens from DNA or RNA, whole antigen-protein, idiotype protein, tumor lysate, whole tumor or viral vector-delivered whole antigen.

7. The dendritic cell vaccine for the use according to claim 6, wherein the antigen source is whole tumor cells, and wherein preferably the tumor cells were killed by high hydrostatic pressure.

8. The dendritic cell vaccine for the use of any one of claims 6 or 7, wherein the whole tumor cells are allogeneic to the patient.

9. The dendritic cell vaccine for the use of any one of claims 1 to 8, wherein the dendritic cells are derived from monocytes obtained by leukapheresis prior to chemotherapy.

10. The dendritic cell vaccine for use of any one of claims 1 to 9, wherein
i. 5 to 30 doses of dendritic cell vaccines are administered and preferentially 8 to 15 doses of dendritic cell vaccines are administered;
ii. the dendritic cell vaccine doses are administered at 1-week intervals, at 2-week intervals, at 3-week intervals, at 5-week intervals or at 6-week intervals; and/or
iii. the dosing schedule comprises 5 initial doses administered during the chemotherapy at 3-week intervals followed by 5 doses at 6-week intervals.

11. The dendritic cell vaccine for use of any one of claims 1 to 9 and 10 option i) or ii),
wherein the dosing schedule comprises 5 doses given at 3-week intervals followed by up to 10 doses at 6-week intervals.

12. The dendritic cell vaccine for use of any one of claims 1 to 11, wherein preferably the ovarian cancer is advanced ovarian cancer, newly diagnosed ovarian epithelial carcinoma or recurrent ovarian cancer.

13. The dendritic cell vaccine for the use of any of the claims 1 to 12, where the chemotherapy treatment comprises a combination treatment selected from a taxane and a platinum complex; gemcitabine and a platinum complex; cisplatin and vinorelbine; cisplatin and etoposide; cisplatin and pemetrexed; cisplatin and irinotecan; gemcitabine and docetaxel; carboplatin and etoposide; carboplatin and pemetrexed and gemcitabine and docetaxel, wherein preferentially:
i. the chemotherapy comprises chemotherapy with a combination of a taxane and a platinum complex, preferably a taxane and a platinum complex selected from paclitaxel and carboplatin; paclitaxel and cisplatin; and docetaxel and carboplatin; wherein more preferentially the combination is paclitaxel and carboplatin; or
ii. the combination is gemcitabine and a platinum complex, wherein preferentially the combination is cisplatin and gemcitabine; or carboplatin and gemcitabine.

## Patentansprüche

1. Impfstoff mit dendritischen Zellen zur Verwendung bei der Behandlung von Krebs, wobei der Impfstoff mit dendritischen Zellen einem Patienten parallel zu einer Chemotherapie mit mindestens einem Chemotherapeutikum verabreicht wird, wobei der Krebs Eierstockkrebs ist und wobei das Chemotherapeutikum ausgewählt ist aus Carboplatin, Cisplatin, Paclitaxel, Docetaxel, Gemcitabin, PEGyliertem liposomalem Doxorubicin, Etoposid, Topotecan, Irinotecan, Olaparib, Rucaparib, Trabectedin, Niraparib, Enzalutamid, Abirateron, Mitoxantron, Cabazitaxel, Vinorelbin, Pemetrexed, Methotrexat, Vinblastin, an Albumin gebundenem Paclitaxel, Erlotinib, Afatinib, Gefitinib, Crizotinib, Alectinib, Ceritinib, Dabrafenib, Trametinib und Osimertinib; sowie Biologika oder einer Kombination daraus.

2. Impfstoff mit dendritischen Zellen zur Verwendung nach Anspruch 1, wobei die erste Dosis des Impfstoffs mit dendritischen Zellen während des Chemotherapiezeitraums verabreicht wird und der Impfstoff mit dendritischen Zellen optional nach Abschluss der Chemotherapie weiter verabreicht wird.

3. Impfstoff mit dendritischen Zellen zur Verwendung nach Anspruch 1 oder Anspruch 2, wobei die Chemotherapie eine Erstlinien-Chemotherapie ist.

4. Impfstoff mit dendritischen Zellen zur Verwendung nach Anspruch 1 oder Anspruch 2, wobei der Patient durch eine vorherige chemotherapeutische Behandlung nicht vom Krebs geheilt wurde.

5. Impfstoff mit dendritischen Zellen zur Verwendung nach einem der Ansprüche 1 bis 4, wobei der Impfstoff mit dendritischen Zellen nach Abschluss der Chemotherapie in Verbindung mit einer Erhaltungstherapie weiter verabreicht wird.

6. Impfstoff mit dendritischen Zellen zur Verwendung nach einem der Ansprüche 1 bis 5, wobei der Impfstoff mit dendritischen Zellen außerhalb des Körpers mit einer Antigenquelle beladen wird, wobei die Antigenquelle vorzugsweise aus tumorassoziiertem Peptid bzw. tumorassoziierten Peptiden, ganzen Antigenen aus DNA oder RNA, ganzem Antigenprotein, Idiotyp-Protein, Tumorlysat, ganzem Tumor oder durch virale Vektoren übertragenem ganzem Antigen ausgewählt ist.

7. Impfstoff mit dendritischen Zellen zur Verwendung nach Anspruch 6, wobei die Antigenquelle ganze Tumorzellen sind und wobei vorzugsweise die Tumorzellen durch hydrostatischen Hochdruck abgetötet wurden.

8. Impfstoff mit dendritischen Zellen zur Verwendung nach Anspruch 6 oder 7, wobei die ganzen Tumorzellen für den Patienten allogen sind.

9. Impfstoff mit dendritischen Zellen zur Verwendung nach einem der Ansprüche 1 bis 8, wobei die dendritischen Zellen aus Monozyten abgeleitet sind, die durch Leukapherese vor der Chemotherapie erhalten werden.

10. Impfstoff mit dendritischen Zellen zur Verwendung nach einem der Ansprüche 1 bis 9, wobei
i. 5 bis 30 Dosen Impfstoff mit dendritischen Zellen verabreicht werden und bevorzugt 8 bis 15 Dosen Impfstoff mit dendritischen Zellen verabreicht werden;
ii. die Dosen Impfstoff mit dendritischen Zellen in einem Abstand von 1 Woche, in einem Abstand von 2 Wochen, in einem Abstand von 3 Wochen, in einem Abstand von 5 Wochen oder in einem Abstand von 6 Wochen verabreicht werden; und/oder
iii. das Dosierungsschema 5 Anfangsdosen umfasst, die während der Chemotherapie in einem Abstand von 3 Wochen verabreicht werden, gefolgt von 5 Dosen in einem Abstand von 6 Wochen.

11. Impfstoff mit dendritischen Zellen zur Verwendung nach einem der Ansprüche 1 bis 9 sowie 10 Option i) oder ii), wobei das Dosierungsschema 5 Dosen umfasst, die in einem Abstand von 3 Wochen gegeben werden, gefolgt von bis zu 10 Dosen in einem Abstand von 6 Wochen.

12. Impfstoff mit dendritischen Zellen zur Verwendung nach einem der Ansprüche 1 bis 11, wobei vorzugsweise
der Eierstockkrebs fortgeschrittener Eierstockkrebs, ein neu diagnostiziertes Eierstock-Epithelkarzinom oder rezidivierender Eierstockkrebs ist.

13. Impfstoff mit dendritischen Zellen zur Verwendung nach einem der Ansprüche 1 bis 12, wobei die chemotherapeutische Behandlung eine Kombinationsbehandlung umfasst, die aus einem Taxan und einem Platin-Komplex; Gemcitabin und einem Platin-Komplex; Cisplatin und Vinorelbin; Cisplatin und Etoposid; Cisplatin und Pemetrexed; Cisplatin und Irinotecan; Gemcitabin und Docetaxel; Carboplatin und Etoposid; Carboplatin und Pemetrexed sowie Gemcitabin und Docetaxel ausgewählt ist, wobei vorzugsweise:
i. die Chemotherapie eine Chemotherapie mit einer Kombination aus einem Taxan und einem Platin-Komplex, vorzugsweise einem Taxan und einem Platin-Komplex, ausgewählt aus Paclitaxel und Carboplatin; Paclitaxel und Cisplatin sowie Docetaxel und Carboplatin umfasst, wobei die Kombination bevorzugter Paclitaxel und Carboplatin ist; oder
ii. die Kombination Gemcitabin und ein Platin-Komplex ist, wobei die Kombination bevorzugt Cisplatin und Gemcitabin oder Carboplatin und Gemcitabin ist.

## Revendications

1. Vaccin à base de cellules dendritiques destiné à être utilisé dans le traitement du cancer, le vaccin à base de cellules dendritiques étant administré à un patient parallèlement à une chimiothérapie avec au moins un agent antinéoplasique, le cancer étant un cancer de l'ovaire, et l'agent antinéoplasique étant sélectionné parmi le carboplatine, le cisplatine, le paclitaxel, le docétaxel, la gemcitabine, la doxorubicine liposomale pégylée, l'étoposide, le topotécan, l'irinotécan, l'olaparib, le rucaparib, la trabectédine, le niraparib, l'enzalutamide, l'abiratérone, la mitoxantrone, le cabazitaxel, la vinorelbine, le pémétrexed, le méthotrexate, la vinblastine, le paclitaxel lié à l'albumine, l'erlotinib, l'afatinib, le géfitinib, le crizotinib, l'alectinib, le céritinib, le dafrafénib, le tramétinib, et l'osimertinib ; et des médicaments biologiques, ou une combinaison de ceux-ci.

2. Vaccin à base de cellules dendritiques destiné à être utilisé selon la revendication 1, dans lequel la première dose du vaccin à base de cellules dendritiques est administrée pendant la période de chimiothérapie, et éventuellement le vaccin à base de cellules dendritiques est en outre administré après l'achèvement de la chimiothérapie.

3. Vaccin à base de cellules dendritiques destiné à être utilisé selon la revendication 1 ou la revendication 2, dans lequel la chimiothérapie est une chimiothérapie de première ligne.

4. Vaccin à base de cellules dendritiques destiné à être utilisé selon la revendication 1 ou la revendication 2, dans lequel un traitement de chimiothérapie précédent n'a pas guéri le patient du cancer.

5. Vaccin à base de cellules dendritiques destiné à être utilisé selon l'une quelconque des revendications 1 à 4, dans lequel le vaccin à base de cellules dendritiques est en outre administré en combinaison avec une thérapie de maintien après l'achèvement de la chimiothérapie.

6. Vaccin à base de cellules dendritiques destiné à être utilisé selon l'une quelconque des revendications 1 à 5, dans lequel le vaccin à base de cellules dendritiques est chargé ex vivo avec une source d'antigène, la source d'antigène étant de préférence sélectionnée parmi un/des peptide(s) associé(s) à la tumeur, des antigènes complets issus d'ADN ou d'ARN, une protéine antigène complète, une protéine idiotypique, un lysat tumoral, une tumeur complète ou un antigène complet délivré par vecteur viral.

7. Vaccin à base de cellules dendritiques destiné à être utilisé selon la revendication 6, dans lequel la source d'antigène consiste en des cellules tumorales complètes, et dans lequel les cellules tumorales ont de préférence été tuées par une pression hydrostatique élevée.

8. Vaccin à base de cellules dendritiques destiné à être utilisé selon l'une quelconque des revendications 6 ou 7, dans lequel les cellules tumorales complètes sont allogéniques pour le patient.

9. Vaccin à base de cellules dendritiques destiné à être utilisé selon l'une quelconque des revendications 1 à 8, dans lequel les cellules dendritiques sont dérivées de monocytes obtenus par leucaphérèse avant la chimiothérapie.

10. Vaccin à base de cellules dendritiques destiné à être utilisé selon l'une quelconque des revendications 1 à 9, dans lequel
i. 5 à 30 doses de vaccin à base de cellules dendritiques sont administrées et de préférence 8 à 15 doses de vaccin à base de cellules dendritiques sont administrées ;
ii. les doses de vaccin à base de cellules dendritiques sont administrées à des intervalles d'1 semaine, à des intervalles de 2 semaines, à des intervalles de 3 semaines, à des intervalles de 5 semaines ou à des intervalles de 6 semaines ; et/ou
iii. le schéma posologique comprend 5 doses initiales administrées pendant la chimiothérapie à des intervalles de 3 semaines suivies de 5 doses à des intervalles de 6 semaines.

11. Vaccin à base de cellules dendritiques destiné à être utilisé selon l'une quelconque des revendications 1 à 9 et 10 option i) ou ii), dans lequel le schéma posologique comprend 5 doses données à des intervalles de 3 semaines suivies de jusqu'à 10 doses à des intervalles de 6 semaines.

12. Vaccin à base de cellules dendritiques destiné à être utilisé selon l'une quelconque des revendications 1 à 11, dans lequel, de préférence,
le cancer de l'ovaire est un cancer avancé de l'ovaire, un carcinome épithélial de l'ovaire nouvellement diagnostiqué ou un cancer récidivant de l'ovaire.

13. Vaccin à base de cellules dendritiques destiné à être utilisé selon l'une quelconque des revendications 1 à 12, dans lequel le traitement de chimiothérapie comprend un traitement combiné sélectionné parmi un taxane et un complexe à base de platine ; de la gemcitabine et un complexe à base de platine ; du cisplatine et de la vinorelbine ; du cisplatine et de l'étoposide ; du cisplatine et du pémétrexed ; du cisplatine et de l'irinotécan ; de la gemcitabine et du docétaxel ; du carboplatine et de l'étoposide ; du carboplatine et du pémétrexed et de la gemcitabine et du docétaxel ; dans lequel, de préférence :
i. la chimiothérapie comprend une chimiothérapie avec une combinaison d'un taxane et d'un complexe à base de platine, de préférence un taxane et un complexe à base de platine sélectionné parmi le paclitaxel et le carboplatine ; le paclitaxel et le cisplatine ; et le docétaxel et le carboplatine ; la combinaison étant plus préférentiellement du paclitaxel et du carboplatine ; ou
ii. la combinaison est de la gemcitabine et un complexe du platine, la combinaison étant de préférence du cisplatine et de la gemcitabine ; ou du carboplatine et de la gemcitabine.
